(19) **Europäisches Patentamt European Patent Office Office européen des brevets**

(11) **EP 3 766 449 B1**

(12) **EUROPEAN PATENT SPECIFICATION**

(45) Date of publication and mention of the grant of the patent:
**24.05.2023 Bulletin 2023/21**

(21) Application number: **19766462.6**

(22) Date of filing: **05.03.2019**

(51) International Patent Classification (IPC):
*A61B 34/37* (2016.01)   *A61B 34/00* (2016.01)
*A61B 34/30* (2016.01)   *A61B 90/00* (2016.01)

(52) Cooperative Patent Classification (CPC):
**A61B 34/76; A61B 34/30;** A61B 90/361;
A61B 2034/302; A61B 2090/064; A61B 2090/065

(86) International application number:
**PCT/CN2019/077059**

(87) International publication number:
**WO 2019/174496 (19.09.2019 Gazette 2019/38)**

(54) **SURGICAL ROBOT SYSTEM AND SURGICAL INSTRUMENT THEREOF**

CHIRURGISCHES ROBOTERSYSTEM UND CHIRURGISCHES INSTRUMENT DAFÜR

SYSTÈME DE ROBOT CHIRURGICAL ET INSTRUMENT CHIRURGICAL ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.03.2018 CN 201810218810**

(43) Date of publication of application:
**20.01.2021 Bulletin 2021/03**

(73) Proprietor: **Shanghai Microport Medbot (Group) Co., Ltd.**
**Shanghai 201203 (CN)**

(72) Inventors:
• **XIA, Yuhui**
  **Shanghai 201203 (CN)**
• **HE, Chao**
  **Shanghai 201203 (CN)**
• **LI, Tao**
  **Shanghai 201203 (CN)**
• **WANG, Jiayin**
  **Shanghai 201203 (CN)**
• **SHI, Yunlei**
  **Shanghai 201203 (CN)**

(74) Representative: **Patentship Patentanwaltsgesellschaft mbH**
**Elsenheimerstraße 65**
**80687 München (DE)**

(56) References cited:
WO-A1-2018/019077     CN-A- 101 340 850
CN-A- 101 578 494      CN-A- 102 151 179
CN-A- 106 264 719      CN-A- 106 618 736
CN-A- 106 618 736      CN-A- 108 433 814
JP-A- 2005 103 056     US-A1- 2007 151 390
US-A1- 2009 157 092    US-A1- 2009 157 092
US-A1- 2009 177 095    US-A1- 2010 094 312
US-A1- 2016 022 373    US-B1- 8 649 847

**Description**

**TECHNICAL FIELD**

The present invention relates to the technical field of medical devices and, more specifically, to a surgical robot system and a surgical instrument thereof.

**BACKGROUND**

A robotically-assisted surgical system usually adopts a master-slave control structure consisting of a master (surgeon side) and a slave (patient side). The master is usually capable of multi-dimensional movement based on components such as robotic arms, multi-dimensional mice, joysticks, etc., while the slave is usually a robotic arm with multiple degrees of freedom holding, at an end thereof, surgical instruments (e.g., needle holders, electrocoagulation forceps, electrosurgical cautery probes, tissue graspers, scissors, etc.) During the operation, based on a 3D image of a target surgical site from an endoscope displayed on a display device, the surgeon operates the master so that the slave follows motion of the master to drive the surgical instruments to perform various surgical operations, e.g., retracting tissue, suturing, grasping a blood vessel, dissecting, cauterizing, coagulating tissue, etc.

Such a robotically-assisted surgical system offers the following advantages:

First, it is designed to perform a minimally invasive intervention through a small surgical incision made in a patient's body, which results in less trauma to the patient and hence reduced postoperative pain, rapid recovery, a lower risk of infection and a minimized need for blood transfusion.

Second, it operates much more precisely and stably by the system when compared to human hands. For example, the system can eliminate physiological tremor, and even tiny capillaries can be clearly viewed in the 3D visual operative field, allowing high surgical safety.

However, since robotically-assisted system is based on tele-operation mode which fails in enabling the surgeon to have a direct tactile sensation, identifying tissue toughness, feeling vascular pulsation, as well as lack of feedback to the gripping and pressing actions. A feedback may occur only when a serious collision to the human body by the surgical instrument occurs, and the sensitive feedbacks are difficult to occur in the case of slight touches. Therefore, imparting to such a robot the ability to adjust motion of the slave according to variation of a force exerted on the terminal end of a surgical instrument that is being brought into contact with a body tissue and to provide the surgeon with appropriate force feedback indications can significantly augment the system's safety.

Most traditional force feedback mechanisms rely on torque sensors disposed at joints of the robot, and a contact torque on the terminal end can be calculated based on the torque on the joints. This approach, however, suffers from a number of disadvantages in that the calculated contact force cannot well reflect in real time what is actually going on. Moreover, the detectability of a minor contact force requires augmenting the torque sensors' measurement accuracy and resolution, which will makes the sensor having a high resolution sensitive to noises, easy to cause signal distortions and result in expensive costs. Further, transmission in the surgical instrument is usually achieved by wires, which are obviously not suitable for arranging tension sensors thereon.

Patent US8491574B2 discloses a solution for the above problems, in which a strain sensitive element capable of directly measuring an external force is embedded in the terminal end of a surgical instrument. This, however, is still associated with the following shortcomings:

First, it is inevitable that a single surgical operation needs a lot of surgical instruments. When each of these surgical instruments is provided with a respective sensor, it will be difficult to ensure measurement consistency across the different sensors. Even when each sensor is calibrated, accurate recording of all the instruments' physical parameters is difficult.

Second, physical properties of the sensitive elements are susceptible to factors such as temperature, humidity and atmospheric pressure, and the performances of the sensitive elements vary between environments such as, inside and outside the patient's body. Therefore, it is difficult for such sensitive elements to describe forces acting on the surgical instruments in a consistent way.

Third, the surgical instruments themselves are consumable and can be generally used for up to 10 times, but the sensors thereon are reusable for more times. Obviously, scraping the surgical instrument together with the sensors is not an economical practice.

Patent publication WO2009079301A1 discloses a force sensor apparatus including an inner tube and an outer tube disposed over the inner tube. Strain gauges are disposed on the inner tube, and a proximal end of the inner tube is connected to a surgical instrument so that the strain gauges on the inner tube can sense a force acting on the surgical instrument. However, this force sensor apparatus is associated with a number of problems such as poorer measurement consistency and lower measurement accuracy.

Patent publication CN104764552A discloses a force-sensitive sensor for sensing surgical forces, which is complicated in structure with multiple beams. In addition, since strain gauges are not directly arranged on components that undergo strains, the sensing accuracy thereof is poorer. Further, this force-sensitive sensor suffers from the following problems: due to the absence of a precise mathematical model, the measurement can rely only on calibration, making it impossible to obtain accurate results; and the axial force measurement is not available due to its structural design.

US 2009/0157092 A1 discloses apparatuses, systems and methods for sensing forces applied to a surgical instrument. In one embodiment, a force sensor includes a tube portion that includes a plurality of radial ribs and a strain gauge positioned over each of the plurality of radial ribs. A proximal part of the tube portion is coupled to a shaft of the surgical instrument that is operably coupled to a manipulator arm of a robotic surgical system. A distal part of the tube portion is coupled to a wrist joint coupled to an end effector. The couplings may be direct or indirect with an intermediate mechanical component between the coupled parts.

US 2007/0151390 A1 discloses apparatuses, systems and methods for improving force and torque sensing and feedback to the surgeon performing a telerobotic surgery, wherein groups of axially oriented strain gauges are positioned on a distal end of an instrument shaft proximate a moveable wrist of a robotic surgical instrument to sense forces and torques at the distal tip of the instrument.

JP 2005103056A discloses a detachable force sensing sleeve for removable attachment to the shaft of a surgical instrument that is configured to measure the radial force applied to the instrument's end effector.

## SUMMARY OF THE INVENTION

It is an objective of the some embodiments of the present invention to provide a surgical robot system and a surgical instrument thereof, which can solve one or more of the above-described problems in the prior art, including low accuracy in measuring a contact force on the terminal end of a surgical instrument and unnecessary scrappage of sensors.

The above and related objectives are achieved by providing a surgical instrument, comprising a mechanical structure, a sleeve and a force sensor;

wherein the mechanical structure comprises a shaft and an end effector connected to a terminal end of the shaft;
the sleeve fixedly or detachably sleeves over the terminal end of the shaft;
the force sensor comprises at least one sensitive element and a peripheral measurement module connected to the sensitive element, the sensitive element is disposed on the sleeve and configured to obtain a strain information of the sleeve;
the peripheral measurement module is configured to obtain a stress measured by the sensitive element based on the strain information obtained by the sensitive element and obtain both an equivalent resultant force and an equivalent resultant moment of an action force exerted on the shaft to the sleeve based on the stress measured by the sensitive element, the position of the sensitive element and the direction of a sensitive axis of the sensitive element, thereby obtaining a contact force acting on the end effector of the surgical instrument.

Optionally, the sleeve comprises a hollowed-out feature extending in a circumferential direction, the hollowed-out feature comprising a plurality of compliant elements and openings each located between adjacent two of the compliant elements, the compliant elements being configured to connect an upper section of the sleeve above the hollowed-out feature to a lower section of the sleeve below the hollowed-out feature, and wherein the at least one sensitive element is arranged on the compliant elements and configured to measure a stress on the compliant elements.

Optionally, the compliant elements have a modulus of elasticity that is not higher than a modulus of elasticity of the shaft.

Optionally, each of the compliant elements comprises an inner surface, an outer surface and side surfaces, and wherein the at least one sensitive element is arranged on the inner surfaces, outer surfaces or side surfaces of the compliant elements.

Optionally, the hollowed-out feature is arranged around an axial middle of the sleeve.

Optionally, the sensitive axis of the sensitive element is arranged to be parallel or perpendicular to an axial direction of the sleeve.

Optionally, the sleeve fixedly sleeves over the terminal end of the shaft by bonding, an interference fit or insertion.

Optionally, the sleeve detachably sleeves over the terminal end of the shaft by thread connection, a latch connection or a snap fitting.

Optionally, the sleeve has one end flush with the terminal end of the shaft.

Optionally, the peripheral measurement module comprises, communicatively connected to one another in series, a data acquisition unit, a signal conditioning unit and a calculation and output unit; wherein the data acquisition unit is configured to acquire signals output from the sensitive element, the signal conditioning unit is configured to condition the signals output from the sensitive element, and the calculation and output unit is configured to perform a calculation based on the conditioned signals to obtain the contact force on the end effector.

Optionally, the stress measured by the sensitive element is given by:

$$f_i = g(\varepsilon_i) = k_i \cdot \varepsilon_i + f(\varepsilon_i) \qquad i = 1, 2, \cdots, n$$

where $i$ represents an i-th sensitive element; $f_i$, a stress measured by the i-th sensitive element; $\varepsilon_i$, a strain of the compliant element, on which the i-th sensitive element is arranged, along the direction of the sensitive axis of the i-th sensitive element; $g(\cdot)$, a function describing a relationship between the stress and the strain; $k_i$, a stress-strain factor of the compliant element, on which the i-th sensitive element is arranged, along the direction of the sensitive axis of the i-th sensitive element; and $f(\varepsilon_i)$, a non-linear compensation term for the stress and the strain.

Optionally, the contact force comprises an equivalent resultant force $\boldsymbol{F}_q$ and an equivalent resultant moment $M_q$ ;

the peripheral measurement module is configured to establish a coordinate system {p} for the sleeve thereon, a coordinate system {q} for the surgical instrument at the terminal end thereof, and descriptors of the position and posture of the coordinate system {p} in the coordinate system {q};
the peripheral measurement module is further configured to obtain a descriptor of the stress measured by the sensitive element in the coordinate system {q} based on the descriptor of the posture of the coordinate system {p} in the coordinate system {q}, a descriptor of the direction of the sensitive axis of the sensitive element in the coordinate system {p} and the stress measured by the sensitive element, thereby obtaining the equivalent resultant force $\boldsymbol{F}_q$ ;
the peripheral measurement module is further configured to obtain a descriptor of the position of the sensitive element in the coordinate system {q} based on the descriptors of the position and posture of the coordinate system {p} in the coordinate system {q} and a descriptor of the position of the sensitive element in the coordinate system {p}; and
the peripheral measurement module is further configured to obtain a descriptor of a moment of force acting on the sleeve in the coordinate system {q} based on the descriptor of the stress measured by the sensitive element in the coordinate system {q}, thereby obtaining the equivalent resultant moment $\boldsymbol{M}_q$ .

Optionally, a descriptor of the stress measured by the sensitive element in the coordinate system {p} is given by:

$$\left(f_{xi}, f_{yi}, f_{zi}\right)^T = f_i \boldsymbol{e}_i = f_i \cdot (e_{xi}, e_{yi}, e_{zi})^T \qquad i = 1, 2, \ldots, n$$

where $\boldsymbol{e}_i$ is a unit vector denoting a descriptor of the direction of the sensitive axis of the i-th sensitive element in the coordinate system {p}; $f_i$, the stress measured by the i-th sensitive element; $f_{xi}, f_{yi}, f_{zi}$, components of the $f_i$ along axes of the coordinate system {p}; and $e_{xi}, e_{yi}, e_{zi}$, components of $e_i$ along axes of the coordinate system {p}.

Optionally, the descriptor of the stress measured by the sensitive element in the coordinate system {q} is given by:

$$f_i = {}^q\boldsymbol{R}\left(f_i \boldsymbol{e}_i\right)$$

where $\boldsymbol{f_i}$ represents a descriptor of the stress measured by the i-th sensitive element in the coordinate system {q}, and ${}^q\boldsymbol{R}$ represents the descriptor of the posture of the coordinate system {p} in the coordinate system {q}, and wherein the equivalent resultant force $\boldsymbol{F}_q$ is given by:

$$\boldsymbol{F}_q = diag(1/n_x, 1/n_y, 1/n_z) \sum_{i=1}^{n} \boldsymbol{f}_i$$

where $diag(\cdot)$ represents a diagonal matrix with elements in the vector $\cdot$ as diagonal elements, and $n_x, n_y, n_z$ represent numbers of redundant forces in the directions of the x-axis, y-axis and z-axis, respectively.

Optionally, the descriptor of the position of the sensitive element in the coordinate system {q} is given by:

$$r_i = r_p + {}^q R \, {}^p r_i$$

where $r_i$ is a descriptor of the position of the i-th sensitive element in the coordinate system {q}; ${}^p r_i$, a descriptor of the position of the i-th sensitive element in the coordinate system {p}; ${}^q R$, the descriptor of the posture of the coordinate system {p} in the coordinate system {q}; and $r_p$, a descriptor of the position of the coordinate system {p} in the coordinate system {q}.

Optionally, the equivalent resultant moment $M_q$ is given by:

$$M_q = diag(1/n_x\,', 1/n_y\,', 1/n_z\,') \sum_{i=1}^{n} r_i \times f_i$$

where $n_x', n_y', n_z'$ represent the numbers of redundant moments in the directions of the x-axis, y-axis and z-axis, respectively.

Optionally, the hollowed-out feature comprises four compliant elements that are arranged along the circumferential direction; the force sensor comprises four sensitive elements, each arranged on an outer surface of a respective one of the compliant elements; the descriptors $e_1$, $e_2$, $e_3$, $e_4$ of the directions of the sensitive axes of the four sensitive elements in the coordinate system {p} are all $(1, 0, 0)^T$ ;

the descriptors of the stress measured by the four sensitive elements in the coordinate system {q} are given as:

$$\begin{cases} f_1 = \begin{pmatrix} f_{x1} & 0 & 0 \end{pmatrix}^T = f_1 e_1 \\ f_2 = \begin{pmatrix} f_{x2} & 0 & 0 \end{pmatrix}^T = f_2 e_2 \\ f_3 = \begin{pmatrix} f_{x3} & 0 & 0 \end{pmatrix}^T = f_3 e_3 \\ f_4 = \begin{pmatrix} f_{x4} & 0 & 0 \end{pmatrix}^T = f_4 e_4 \end{cases}$$

where $f_{x1}$, $f_{x2}$, $f_{x3}$, $f_{x4}$ respectively represent stress measured by the first to fourth sensitive elements in the direction of an x-axis of the coordinate system {q}; and
the equivalent resultant force $F_q$ is given by:

$$F_q = (f_1 + f_2 + f_3 + f_4)\ .$$

18. The surgical instrument of claim 17, wherein:

${}^q R$, the descriptor of the posture of the coordinate system {p} in the coordinate system {q}, is an identity matrix; and
the positions of the sensitive elements are measured as respective center positions, which are described in the coordinate system {q} as:

$$\begin{cases} r_1 = \begin{pmatrix} x_1 & y_1 & z_1 \end{pmatrix}^T = r_p + {}^p r_1 \\ r_2 = \begin{pmatrix} x_2 & y_2 & z_2 \end{pmatrix}^T = r_p + {}^p r_2 \\ r_3 = \begin{pmatrix} x_3 & y_3 & z_3 \end{pmatrix}^T = r_p + {}^p r_3 \\ r_4 = \begin{pmatrix} x_4 & y_4 & z_4 \end{pmatrix}^T = r_p + {}^p r_4 \end{cases}$$

where ${}^p r_1$, ${}^p r_2$, ${}^p r_3$, ${}^p r_4$ are descriptors of the center positions of the four sensitive elements in the coordinate system {p}; ${}^r p$, the descriptor of the position of the coordinate system {p} in the coordinate system {q}; $x_1, x_2, x_3, x_4$, x-axis coordinates of the center positions of the first to fourth sensitive elements in the coordinate system {q}; $y_1, y_2, y_3, y_4$, y-axis coordinates of the center positions of the first to fourth sensitive elements in the coordinate system {q};

$z_1$, $z_2$, $z_3$, $z_4$, z-axis coordinates of the center positions of the first to fourth sensitive elements in the coordinate system {q}; and $r_1$, $r_2$, $r_3$, $r_4$, descriptors of the positions of the first to fourth sensitive elements in the coordinate system {q}.

Optionally, the equivalent resultant moment $M_q$ is given as:

$$M_q = \left( r_1 \times f_1 + r_2 \times f_2 + r_3 \times f_3 + r_4 \times f_4 \right).$$

Further, the present invention also provides a surgical robot system, comprising a slave, the slave comprising: a robotic arm; and the surgical instrument as defined above, the robotic arm comprising a terminal end detachably connected to the surgical instrument, the robotic arm being configured to drive the surgical instrument to move about a remote center of motion.

Optionally, the surgical robot system further comprises a master and a control unit, the master comprising a force indicator;

wherein the control unit is communicatively connected to both the master and the slave; the control unit is configured to obtain an information about contact force acting on the end effector from the force sensor of the surgical instrument and transmit the information to the force indicator, the force indicator is configured to show the information about the contact force acting on the end effector.

Optionally, the slave further comprises:

an endoscope; and
an endoscope holder detachably connected to the endoscope;
wherein the peripheral measurement module of the surgical instrument is configured to establish a coordinate system {p} for the sleeve, a coordinate system {q} for the surgical instrument and a coordinate system {e} for the endoscope, and to obtain, from a matrix ${}^eR$ describing a rotation from the coordinate system {q} to the coordinate system {e} and a position vector ${}^er = (r_x\ r_y\ r_z)^T$ from the coordinate system {q} to the coordinate system {e}, a descriptor of the contact force acting on the end effector in the coordinate system {e} as:

$$\begin{pmatrix} {}^eF \\ {}^eM \end{pmatrix} = \begin{pmatrix} {}^eR & 0 \\ -S^T({}^er){}^eR & {}^eR \end{pmatrix} \begin{pmatrix} F_q \\ M_q \end{pmatrix}$$

where ${}^eF$ denotes a resultant force in the coordinate system {e}; ${}^eM$, a resultant moment in the coordinate system {e}; $S({}^er)$, an anti-symmetric matrix corresponding to the vector ${}^er$; $F_q$, the equivalent resultant force of the contact force acting on the end effector; $Mq$, the equivalent resultant moment of the contact force acting on the end effector; and

$$S({}^er) = \begin{pmatrix} 0 & -r_z & r_y \\ r_z & 0 & -r_x \\ -r_y & r_x & 0 \end{pmatrix}.$$

In summary, the surgical robot system and the surgical instrument thereof provided in the present invention provide at least one of the following advantages:

First, no clearance occurs between the sleeve and the mechanical structure of the surgical instrument. This is conducive to improved measurement accuracy.

Second, for a surgical operation requiring the use of several surgical instruments, a specific calibration process can be performed on each of the surgical instruments, avoiding the influence of instrument material inconsistency on the measurement.

Third, identical sleeve can be used on different surgical instruments which transmits deformations of these surgical instruments. Compared to sensitive elements directly attaching to the terminal ends of the surgical instruments, the use of the sensitive elements with the sleeve can avoid measurement errors that may arise from measurement inconsistency between the different sensitive elements on the different surgical instruments, helping in achieving a higher degree of measurement precision and accuracy.

Fourth, coupling the sensitive elements to the surgical instruments with the sleeves can avoid scrappage of the sensitive elements together with the surgical instruments. The surgical instruments are consumable, while the sensitive elements are reusable. Obviously, scraping the sensitive elements together with the surgical instruments is not an economic practice and will lead to increased usage cost.

In a preferred embodiment of the present invention, a plurality of sensitive elements are arranged on the compliant elements of the sleeve in a predetermined manner, and each of the sensitive elements has predetermined coordinates in the coordinate system for the surgical instrument. Based on the predetermined coordinates, the positions of the sensitive elements on the compliant elements can be determined, thus allowing one or more dimensional measurement of a contact force acting on the terminal end of the surgical instrument. This entails an easier and simpler contact force measurement approach, in which one or more dimensional contact force measurement is achievable only by adjusting the positions of the sensitive element relative to the coordinate system for the surgical instrument.

## BRIEF DESCRIPTION OF THE DRAWINGS

Fig. 1 shows a schematic of a surgical robot system according to an embodiment of the present invention.
Fig. 2 is a schematic diagram illustrating the mechanical structure of a surgical instrument according to an embodiment of the present invention.
Fig. 3 schematically illustrates the surgical instrument that is performing a surgical operation according to an embodiment of the present invention.
Fig. 4a is a schematic diagram of a force sensor and a sleeve in the surgical instrument according to an embodiment of the present invention.
Fig. 4b is a structural block diagram of a peripheral measurement module in the surgical instrument according to an embodiment of the present invention.
Fig. 5a is a schematic diagram of a sleeve disposed over a shaft in the surgical instrument according to an embodiment of the present invention.
Fig. 5b is a schematic enlarged partial view of the sleeve and the shaft of Fig. 5a.
Fig. 6a is a schematic diagram of the sleeve that has not been assembled with the shaft according to an embodiment of the present invention.
Fig. 6b is a schematic diagram of the sleeve that has not been assembled with the shaft according to another embodiment of the present invention.
Fig. 7a is a schematic diagram of the surgical instrument that is being stressed on one side and deformed according to an embodiment of the present invention.
Fig. 7b is a schematic diagram illustrating analysis on a force acting on the terminal end of the surgical instrument and measurement principle thereof in accordance with an embodiment of the present invention.
Fig. 8 is a schematic diagram illustrating force measurement principles of a three-dimensional force sensor according to an embodiment of the present invention.

In these figures,

1 denotes a surgical cart; 2, a robotic arm; 3, a surgical instrument; 301, a power module; 302, a shaft; 303, an end effector; 304, a roll joint; 305, a pitch joint; 306, 306', a yaw joint; 4, an endoscope; 5, an imaging system; 6, a surgeon; 7, a master manipulator; 8, a patient;
400, a force sensor; 401, a sleeve; 4011, a compliant element; 402, a sensitive element; 403, a peripheral measurement module; 4031, a data acquisition unit; 4032, a signal conditioning unit; and 4033, calculation and output unit.

## DETAILED DESCRIPTION

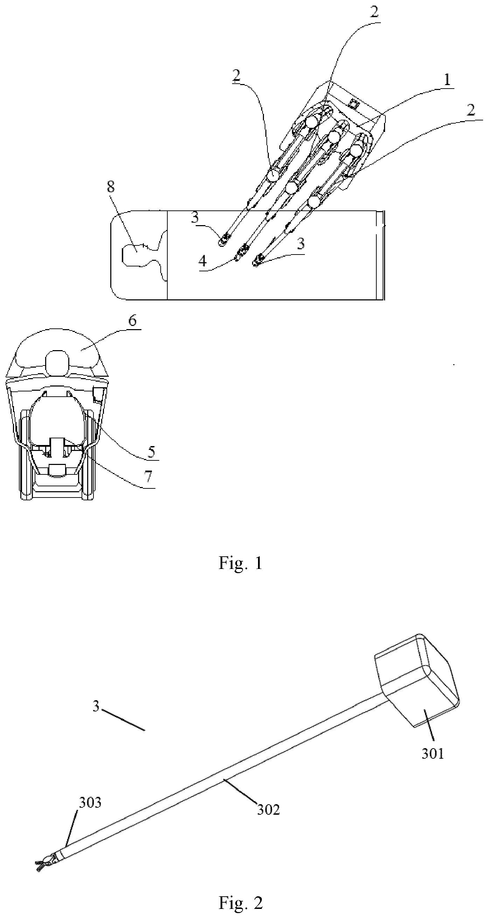

Objectives, advantages and features of the present invention will become more apparent upon reading the following description of the surgical robot system and the surgical instrument thereof proposed in the invention in conjunction with the accompanying drawings, i.e., Figs. 1 through 8. Note that the figures are provided in a very simplified form not necessarily presented to scale, with their only intention to facilitate convenience and clarity in explaining a few embodiments disclosed herein. As used herein, a "terminal end" or "distal end" of a product refers to the end thereof farther away from a user operating the product, while a "proximal end" refers to the end of the product closer to the user.
Fig. 1 is a schematic of a surgical robot system according to an embodiment of the present invention. As shown in Fig. 1, the surgical robot system includes a slave including a surgical cart 1, robotic arms 2, a surgical instrument 3 and an endoscope 4. The surgical cart 1 serves as a base of the slave and supports all mechanical components thereof. Preferably, the surgical cart 1 is able to move on the ground so as to bring the slave closer to or away from a patient 8.

The robotic arm 2 is mounted on the surgical cart 1 and has multiple degrees of freedom, which make it able to move within a certain spatial range. When the surgical cart 1 moves into the vicinity of the patient 8, the robotic arm 2 can be adjusted to place the surgical instrument 3 at a planned operating position. The surgical instrument 3 is detachably mounted at a terminal end of one of the robotic arms 2 and is configured as an actuating mechanism of the slave, which is able to pivot about a remote center of motion (RCM) under the drive of the robotic arm 2. The surgical instrument 3 has an end effector 303 configured to be inserted into the patient's body to treat a lesion therein.

The endoscope 4 is mounted at a terminal end of another robotic arm 2 (which is therefore also referred to herein as an "endoscope holder") and is configured to collect image information in the surgical environment, including but not limited to information about the lesion and information about the position and posture of the surgical instrument 3. Additionally, the endoscope 4 mounted on the robotic arm 2 is communicatively connected to the master as detailed below configured to display in real time the collected image information in the surgical environment. The endoscope 4 may be stereoscopic or not, without limitation.

Fig. 2 is a schematic diagram illustrating the mechanical structure of a surgical instrument according to an embodiment of the present invention. As shown in Fig. 2, the mechanical structure of the surgical instrument 3 includes a power module 301, a shaft 302, a transmission mechanism and an end effector 303. The transmission mechanism may be a wire transmission mechanism that is housed in the shaft 302 and coupled to both the power module 301 and the end effector 303. The power module 301 may be disposed at a proximal end of the shaft 302, and the end effector 303 may be disposed at a distal end of the shaft 302.

The power module 301 is configured to provide the end effector 303 with a driving force, which is transmitted to the end effector 303 through the transmission mechanism and configured to cause the end effector 303 to perform a multi-dimensional movement such as pivoting, opening/closing, etc. Specific opreations that the end effector 303 can perform on the lesion in the patient may include cutting, probing, grasping, etc. Accordingly, the end effector 303 may be implemented as a scissor, a forcep, a probe, etc. The power module 301 may be self-powered. In this case, for example, the power module 301 may include a power source and a driver. Alternatively, the power module 301 may also be externally powered so as to provide the end effector 303 with a driving force. In this case, for example, the power module 301 may include an interface connected to an external power supply and, preferably, may further include a power distribution module.

As shown in Fig. 1, the surgical robot system further includes a master including an imaging system 5 and a master manipulator 7. During a surgical operation, while monitoring the movement of the surgical instrument 3 that is indicated by image information collected by the endoscope 4 and displayed on the imaging system 5, the surgeon 6 can control the surgical instrument 3 to perform multi-dimensional movements as necessary for the operation, such as pitching, yawing, rotating, opening/closing, etc., by manipulating the master manipulator 7.

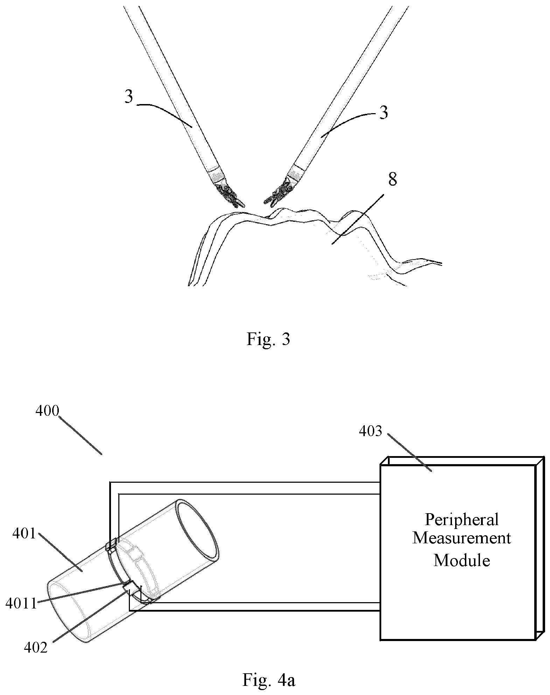

Fig. 3 schematically illustrates a surgical instrument performing surgical operations according to an embodiment of the present invention that is performing a surgical operation. As shown in Fig. 3, the surgeon 6 can manipulate one or more such surgical instruments 3 (identical or not) through the master manipulator 7 to perform cutting, probing, grasping and other actions as required by a surgical operation.

Obviously, the control of master manipulator 7 over the surgical instrument(s) 3 is the basis for master-slave control of the surgical robot system. However, since such control is based on tele-operation, the surgeon cannot directly feel the magnitude of an applied force, which is not favorable for the surgeon to take an appropriate action based on the strength of a contact force. To overcome this, the present invention provides a surgical instrument with force feedback capabilities, which allow the surgeon 6 to feel a force acting on a terminal end of the surgical instrument.

Fig. 4a is a schematic diagram of a force sensor and a sleeve in the surgical instrument according to an embodiment of the present invention. As shown in Fig. 4a, in addition to the above-discussed mechanical components, the surgical instrument 3 further includes the sleeve 401 and the force sensor 400. The force sensor 400 includes sensitive elements 402 and a peripheral measurement module 403. The sleeve 401 includes a hollow lumen with an inner diameter that matches an outer diameter of the shaft 302 in the surgical instrument 3. Further, the sleeve 401 includes a hollowed-out feature extending circumferentially which forms a closed circumferential ring across the sleeve 401. Here, the "circumferential extension" of the hollowed-out feature across the sleeve 401 means that a middle cross-section of the hollowed-out feature may be either perpendicular to or inclined with respect to an axis of the sleeve 401. The hollowed-out feature includes a plurality of compliant elements 4011 spaced from one another by openings each between adjacent two of the compliant elements 4011. As such, the compliant elements 4011 couple an upper section of the sleeve (above the hollowed-out feature) to a lower section thereof (below the hollowed-out feature). With this design, an external force is transmitted from one end of the sleeve to the other end via the compliant elements 4011, the magnitude and direction of the external force can be determined by measuring a force transmitted by the respective compliant elements 4011. This design can facilitate external force measurement with reduced algorithm complexity and increased accuracy.

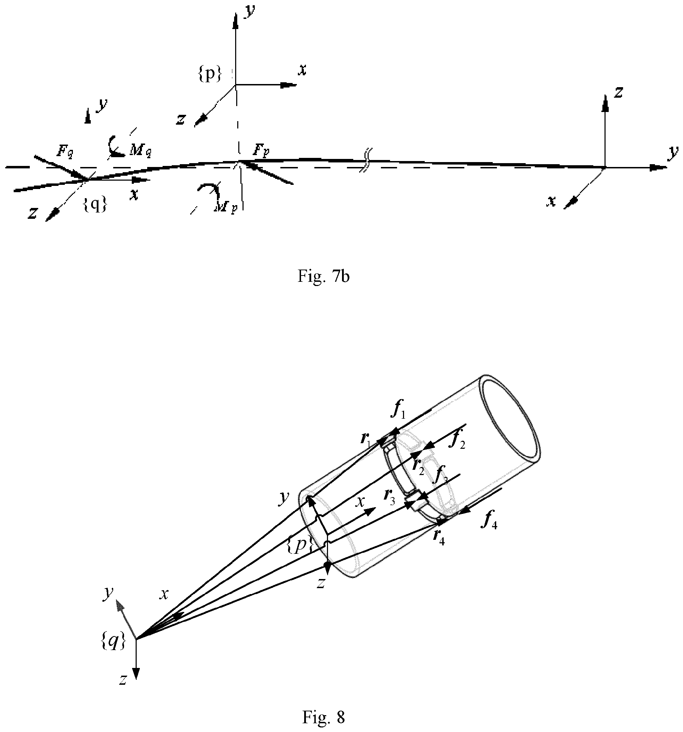

In the preferred embodiment shown in Fig. 8, the hollowed-out feature that extends circumferentially on the sleeve 401 includes four compliant elements 4011 distributed in a centrally symmetric manner and form a cross shape. The present invention is not limited to any particular method for fabricating the hollowed-out feature and the compliant elements 4011. In an exemplary embodiment, the sleeve 401 with the hollowed-out feature is fabricated by removing undesired portions from a tubular blank mechanically, e.g., by wire cutting or electric sparking. In an alternative embodiment, the sleeve may be fabricated by fixing the compliant elements 4011 to the upper and lower sections. Therefore, the compliant elements 4011 may be made either of the same material as, or a different material from, the remainder of the sleeve. In order for improved force measurement sensitivity to be achieved, which can facilitate the sensitive elements 402 to sense an external force acting on the surgical instrument, the compliant elements 4011 may be made of a material selected to have a modulus of elasticity not higher than that of the shaft 302, such as rubber.

Each compliant element 4011 includes an inner surface, an outer surface and side surfaces. The sensitive elements 402 may be disposed to the inner, outer or side surfaces of the compliant elements 4011 in the sleeve 401. Preferably, each of the sensitive elements 402 has a sensitive axis extending in the axial or circumferential direction of the sleeve 401 and can sense the magnitudes of forces acting on the compliant elements 4011 along the sensitive axis. Here, the extension direction of the "sensitive axis" refers to the only direction in which the sensitive elements 402 are sensitive to strain. That is, the sensitive elements 402 are insensitive in any other direction. In some preferred embodiments of the present invention, the sensitive axis of the sensitive element 402 is set to be parallel to the axial direction of the sleeve 401. In practice, the sensitive elements 402 may be so mounted that their sensitive axis extends parallel to the axial direction of the sleeve 401, allowing them to sense strains of the compliant elements 4011 in this direction.

The sensitive elements 402 may be selected as strain gauges, fiber optics or other components, according to their application conditions and measurement requirements. The number of the sensitive elements 402 may be selected, and their arrangement may be designed, in a flexible manner depending on the type of contact force to be measured and the reliability of the sensor. The sensitive elements 402 may be attached on the inner, outer or side surfaces of the compliant elements 4011 in the sleeve 401 in a flexible manner depending on how the attachment is accomplished and on whether it is difficult. Preferably, the hollowed-out feature and hence the compliant elements 4011 are arranged in a portion of the sleeve 401 tending to experience a greater strain (such as an axial middle of the sleeve 401). Preferably, the plurality of sensitive elements 402 arranged on the compliant elements 4011 are capable of one- or multi-dimensional contact force measurement, e.g., three- or six-dimensional contact force measurement. More preferably, each of the compliant elements 4011 is provided (e.g., on the outer surface thereof) with one sensitive element 402.

It is to be noted that structural strength of the sleeve 401 should be taken into account in its practical design. For example, although it is theoretically feasible for the hollowed-out feature to include only one compliant element 4011, structural strength of the sleeve 401 in this case may be too weak to allow it to transmit an external force in a desirable way or to prevent significant plastic deformations of the compliant element 4011, which are detrimental to measurement accuracy. Therefore, the embodiment of Fig. 8 with four compliant elements 4011 that are arranged equiangularly on the circumference of the hollowed-out feature and thus at the four ends of a symmetric cross is preferred.

The peripheral measurement module 403 is communicatively (e.g., electrically) connected to the sensitive element 402 and configured to collect strain information of the compliant elements 4011 obtained by the sensitive elements 402 (possibly in the form of electric signals such as resistive or voltage) and ultimately derive a contact force acting on the terminal end of the surgical instrument from the strain information. In this embodiment, the peripheral measurement module 403 may include a microprocessor for receiving the electric signals output from the sensitive elements 402 and computationally deriving the contact force acting on the terminal end of the surgical instrument based on the received electric signals. In place of the microprocessor, any other programmable computing device such as a single chip microcomputer (SCM), PLC controller, an FPGA or the like may be selected. Optionally, the peripheral measurement module 403 is fixedly connected to the power module 301.

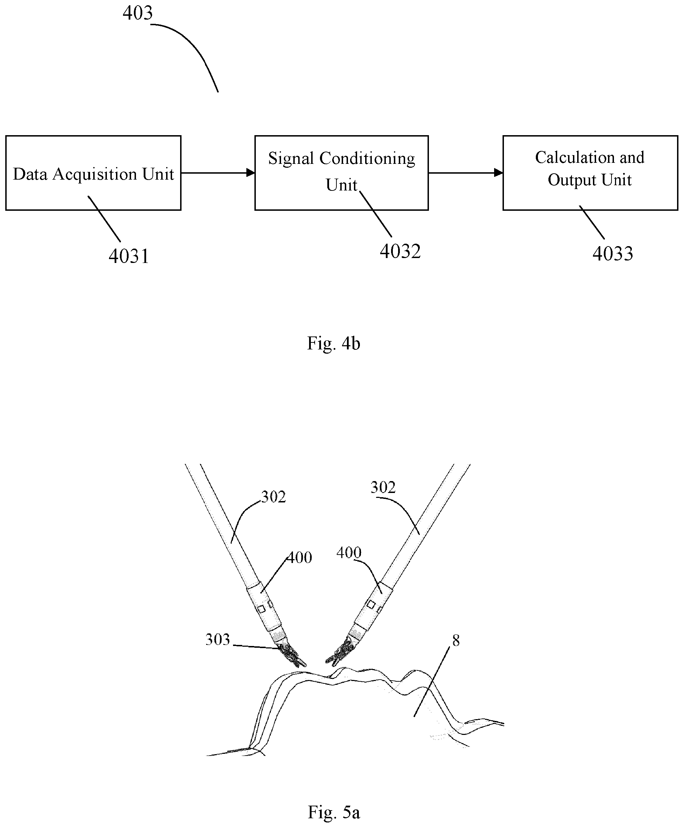

Fig. 4b is a structural block diagram of a peripheral measurement module in the surgical instrument according to an embodiment of the present invention. As shown in Fig. 4b, the peripheral measurement module 403 may include, connected to one another in series, a data acquisition unit 4031, a signal conditioning unit 4032 and a calculation and output unit 4033. Specifically, the peripheral measurement module 403 may operate according to the process: the data acquisition unit 4031 first obtains electric signals generated by the sensitive elements 402; the signal conditioning unit 4032 may then condition the electric signals obtained by the data acquisition unit 4031 through subjecting them to a series of processes including, but not limited to, amplification, digital filtering, detrending, removal of outliers, etc, the calculation and output unit 4033 may at last derive the contact force acting on the terminal end of the surgical instrument 3 by analyzing and calculating the conditioned electric signals from the signal conditioning unit 4032 and output the contact force that may be either one-dimensional or multi-dimensional.

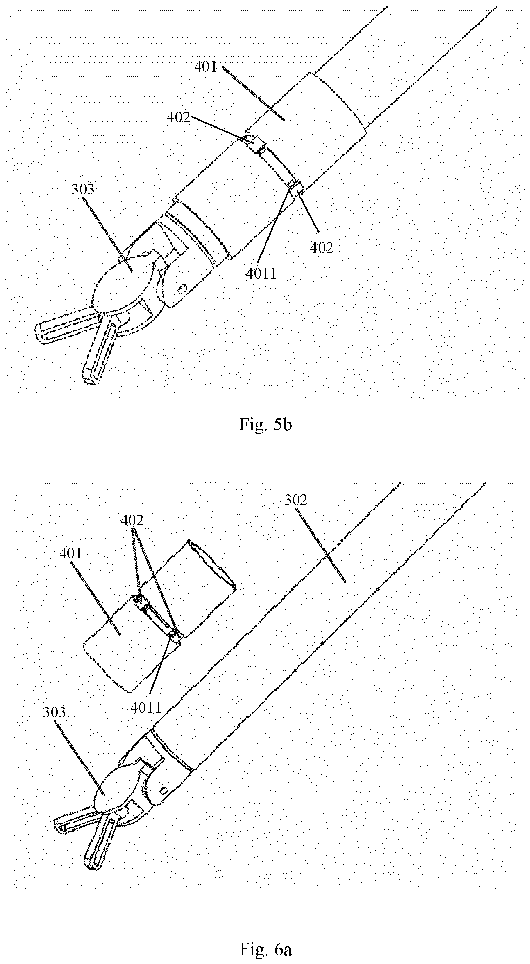

Fig. 5a is a schematic diagram of the sleeve 401 disposed over the shaft 302 in the surgical instrument according to an embodiment of the present invention, and Fig. 5b is a schematic enlarged partial view of the sleeve 401 and the shaft 302 of Fig. 5a. As shown in Figs. 5a and 5b, with the aid of the sleeve 401, the force sensor 400 sleeves over the terminal end of the shaft 302 in the surgical instrument 3. The closer the sleeve 401 is positioned to the end effector 303, the easier the force acting on the terminal end of the surgical instrument 3 can be known. Preferably, the sleeve 401 is flush with an end face of the terminal end of the shaft 302 and connected to the terminal end fixedly by means of an interference fit, insertion, bonding or the like. Alternatively, in order for easier assembly or disassembly to be achieved, it may be connected to the terminal end detachably by means of thread connection, a latch connection, a snap fitting or the like.

Fig. 6a is a schematic diagram of the sleeve 401 that has not been assembled with the shaft 302 according to an embodiment of the present invention. As shown in Fig. 6a, in this embodiment, the sleeve 401 may be optionally disposed fixedly over the shaft 302 by an interference fit. In this case, the sensitive elements 402 are not limited to being disposed on the outer surfaces of the compliant elements 4011, as they can also be disposed on the inner surfaces of the compliant elements 4011 (e.g., in recesses formed in the inner surfaces of the compliant elements 4011). Obviously, in this embodiment, the interference fit that fixedly connects the sleeve 401 to the shaft 302 can be achieved by the elasticity of the sleeve 401 itself.

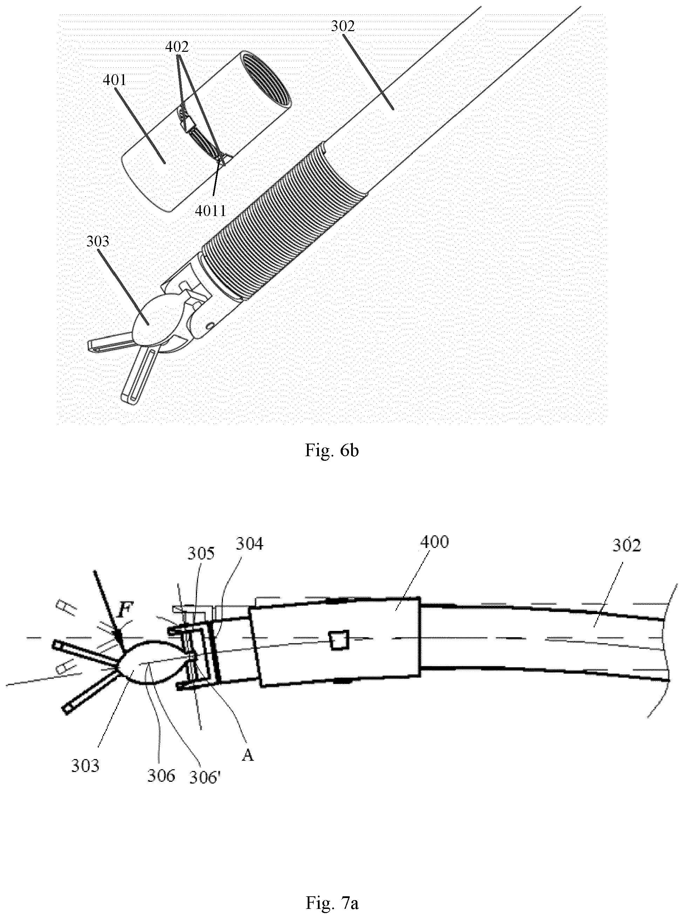

Fig. 6b is a schematic diagram of the sleeve 401 that has not yet been assembled with the shaft 302 according to another embodiment of the present invention. As shown in Fig. 6b, in this embodiment, the sleeve 401 may be optionally disposed detachably over the shaft 302 by a threaded fit, with the sensitive elements 402 preferably arranged on the outer surfaces of the compliant elements 4011. In particular, an internal thread may be provided on an inner surface of the sleeve 401, which can make a threaded fit with an external thread formed in an outer surface of the shaft 302. Such a threaded connecting is advantageous in ensuring the absence of any clearance between the sleeve 401 and the shaft 302, which may exert an adverse impact on force measurement accuracy. In case of threaded connecting, in order to ensure reliability of the connecting, the sleeve 401 is preferably made of a material with high hardness, such as carbon fibers.

Fig. 7a is a schematic diagram of the surgical instrument that is being stressed on one side and deformed according to an embodiment of the present invention. In this embodiment, the surgical instrument 3 successively includes a roll joint 304, a pitch joint 305 and a yaw joint 306, which allow roll (rotation on its own axis), pitch and yaw motions of the end effector 303, respectively. In this embodiment, the end effector 303 includes two flaps (therefore, the end effector is, for example, scissors). Accordingly, two yaw joints (306, 306') are required to control a corresponding one of the flaps respectively. As shown in Fig. 7a, upon the end effector 303 being brought into contact with a body tissue, the body tissue will exert a contact force $F$ on the end effector 303. Apparently, this contact force $F$ will be transmitted by the shaft 302 to one end of the sleeve 401 and further to the other end thereof via the compliant elements 4011. Under the action of the contact force $F$, the compliant elements 4011 will deform as shown in Fig. 7a, and the deformation will be partially sensed by the sensitive elements 402.

Fig. 7b is a schematic diagram illustrating analysis on the force acting on the terminal end of the surgical instrument and measurement principle thereof in accordance with an embodiment of the present invention. As shown in Fig. 7b, when the terminal end of the surgical instrument 3 is brought into contact with a body tissue, the body issue will exert a contact force $F$ on the surgical instrument 3, which may be equivalent to a spatial resultant force $F_q$ and a spatial resultant moment $M_q$, which cause the shaft 302 to deform, together with the sleeve 401, as shown in Figs. 7a and 7b. At the same time, the surgical instrument 3 is subject to a reaction force ($F$p and $M$p) from the sleeve 401. Here, the equivalent spatial resultant force $F_q$ is balanced with $F_p$, and the equivalent resultant moment $M_q$ is balanced with $M$p. The reaction force that the sleeve 401 applies to the surgical instrument 3 can be obtained by measuring the action force exerted by the surgical instrument 3 to the sleeve 401, and the contact force $F$ exerted by the body tissue on the surgical instrument 3 can be derived from force and moment equilibrium equations.

It will be understood that, in Fig. 7b, which schematically illustrates how the terminal end of the surgical instrument is stressed and deformed, a coordinate system {q} may be defined for the surgical instrument at the terminal end thereof, with an origin located, for example, at the point A marked in the figure, i.e., the intersection of an axis of the shaft 302 and an axis of rotation of the pitch joint 305 in the surgical instrument. The coordinate system {q} also has an x-axis that is perpendicular to the axis of rotation of the pitch joint 305 and coincides with the axis of the shaft 302 in an initial configuration, a y-axis extending parallel to the axis of rotation of the pitch joint 305 in the surgical instrument, and a z-axis that is perpendicular to both the above axes and determined by the right-hand rule. Here, the "initial configuration" is defined as a configuration in which the end effector 303 of the surgical instrument 3 extends straight along the direction of the axis of the shaft 302, with its distal end located farthest away from the yaw joint. The origin of the coordinate system {q} may be the point of application of the equivalent resultant force $F_q$ and equivalent resultant moment $M$q. Since this embodiment is not limited to any particular orientation or position of the sensitive elements 402 on the compliant elements 4011, a coordinate system {p} is desired to be defined for the sleeve 401 as a reference serving to facilitate determining the positions and orientations of the sensitive elements 402, with an origin located for example, at the center of an end face thereof, an x-axis extending along an axis of the sleeve 401, and y- and z-axes being perpendicular to each other and both extending radially. Similarly, the origin of the coordinate system {p} may be the point of application of the resultant force (as well as the associated moment) acting on the sleeve. In addition, $^qR$ denotes the transformation

from the coordinate system {p} to {q}, i.e., a descriptor of the posture of the coordinate system {p} in {q}. Since the point of force applied from the surgical instrument 3 to the sleeve 401 is not at the terminal end of the surgical instrument, a position vector $r_p$ is defined in this embodiment to describe the position of the point of application of the resultant force exerted by the surgical instrument 3 on the sleeve 401 in the coordinate system {q}. That is, it is a descriptor of the position of the coordinate system {p} in {q}. Both the transformation matrix $^qR$ and position vector $r_p$ are known quantities that have been determined during assembly. For the sake of simple computation, some means are desirably used during assembly to ensure that the coordinate systems {q} and {p} have the same initial orientation, where $^qR$ is an identity matrix, based on which the coordinate systems according to this embodiment are established. Those skilled in the art will appreciate that the positions and orientations of the coordinate systems {q} and {p} can be flexibly selected to meet the actual requirements.

When the sleeve 401 deforms after receiving an action force ($-F_p$ and $-M_p$) from the shaft 302, the sensitive elements 402 on the compliant elements 4011 may sense deformations of the compliant elements 4011. The deformations of the compliant elements 4011 and the stress measured by the sensitive elements 402 follow the following relation:

$$f_i = g(\varepsilon_i) = k_i \cdot \varepsilon_i + f(\varepsilon_i) \qquad i = 1, 2, \cdots, n \qquad (1\text{-}1)$$

In equation (1-1), $i$ represents the i-th sensitive element out of a total of, for example, $n$ sensitive elements according to this embodiment; $f_i$ denotes a stress measured by the i-th sensitive element; $\varepsilon_i$, the strain of the corresponding compliant element 4011, on which the i-th sensitive element is arranged, along the direction of the sensitive element's sensitive axis; $g(\cdot)$, a functional relation between the stress and the strain; $k_i$, a stress-strain factor of the corresponding compliant element 4011 along the direction of the sensitive axis of the i-th sensitive element, which is determined by material parameters of the compliant element 4011; $f(\varepsilon_i)$, a non-linear compensation item for the stress and the strain, which is obtained by calibration. An exemplary calibration method involves: (i) determining whether there is a zero point drift in any of the sensitive elements 402 by checking signals from the sensitive elements 402 when the sleeve 401 is free of any external force; (ii) determining whether the strain of each sensitive element 402 varies non-linearly with an external force load on the sleeve 401 and, if so, including the non-linear compensation term in the above equation; and (iii) ensuring alignment between directions of the calculated force and actually-applied force. Obviously, as used here, the "stress measured by the sensitive element 402" should not be construed as a direct stress reading of the sensitive element 402. Rather, it refers to a stress on a corresponding portion of the sleeve (i.e., the corresponding compliant element 4011) derived according to the above equation (1-1) from a strain of the portion measured by the sensitive element 402.

In addition, information of the force exerted by the shaft 302 on the sleeve 401 contains its direction, which is a representation of the direction of the sensitive axis of the i-th sensitive element in the coordinate system {p}, and the position of its point of application, which is a representation of the position of the i-th sensitive element in the coordinate system {p}. The stress measured by the sensitive element 402, i.e., the action force exerted by the shaft 302 on the compliant element 4011 is represented in the coordinate system {p} as:

$$\left(f_{xi}, f_{yi}, f_{zi}\right)^T = f_i e_i = f_i \cdot \left(e_{xi}, e_{yi}, e_{zi}\right)^T \qquad (1\text{-}2)$$

In equation (1-2), $e_i$ is a unit vector denoting the representation of the direction of the sensitive axis of the i-th sensitive element (i.e., the direction along which the sensitive element 402 is arranged) in the coordinate system {p}; $f_i$, the stress measured by the i-th sensitive element; $f_{xi}, f_{yi}, f_{zi}$, components of $f_i$ along the axes of the coordinate system {p}; $e_{xi}, e_{yi}, e_{zi}$, components of $e_i$ along the axes of the coordinate system {p}; and $(\cdot)^T$, a matrix transposition operator.

Further, the direction and position of point of application of the force exerted by the shaft 302 to the sleeve 401 can be represented in the coordinate system {q} as:

$$f_i = {}^qR\left(f_i e_i\right) \qquad (1\text{-}3)$$

$$r_i = r_p + {}^qR\,{}^pr_i \qquad (1\text{-}4)$$

In equation (1-3), $e_i$ denotes a representation of the direction along which the i-th sensitive element 402 is arranged (i.e., the direction of the sensitive axis thereof) in the coordinate system {p}; $f_i$, a representation of the stress measured by the i-th sensitive element 402 in the coordinate system {q}; and $^qR$, a representation of the posture of the coordinate system {p} in the coordinate system {q}.

In equation (1-4), $^pr_i$ denotes a representation of the position of the i-th sensitive element (i.e., the position of the point of application) in the coordinate system {p}; $r_i$, a representation of the position of the i-th sensitive element in the coordinate system {q}; and $r_p$, a representation of the position of the coordinate system {p} in the coordinate system {q}.

It is to be noted that, in order to ensure a relative position relationship between the sleeve 401 and the shaft 302 of the surgical instrument and hence between the coordinate systems {p} and {q}, grooves or lines can be engraved or drawn on the end face or surface of the sleeve or on the surface of the shaft 302 to facilitate alignment during assembly.

The stresses measured by all the sensitive elements can be combined in the coordinate system {q} to result in:

$$\begin{pmatrix} F_q \\ M_q \end{pmatrix} = \begin{pmatrix} diag(1/n_x, 1/n_y, 1/n_z)\sum_{i=1}^{n} f_i \\ diag(1/n_x', 1/n_y', 1/n_z')\sum_{i=1}^{n} r_i \times f_i \end{pmatrix} \qquad n_x, n_y, n_z, n_x', n_y', n_z' \geq 1$$

$$(1\text{-}5)$$

As can be seen from equation (1-5), the equivalent resultant force of the contact force acting on the terminal end of the surgical instrument is $F_q = diag(1/n_x, 1/n_y, 1/n_z)\sum_{i=1}^{n} f_i$ , and the equivalent resultant moment thereof is $M_q = diag(1/n_x', 1/n_y', 1/n_z')\sum_{i=1}^{n} r_i \times f_i$ , where $diag(\bullet)$ represents a diagonal matrix with elements in the vector • as its diagonal elements, $n_x, n_y, n_z$ denote the numbers of redundant forces in the x, y, z directions, respectively, and $n_x', n_y', n_z'$ are the numbers of redundant moments in the x, y, z directions, respectively.

In order to improve measurement accuracy and reliability, redundancy, i.e., repeated measurement cycles that are deliberately added, may be sometimes introduced into measurement methods used in practical engineering applications.

The number of redundant forces along a certain direction can be interpreted as the number of times the force is measured by a force sensor along the transmission path. Similarly, the number of redundant moments in a certain direction can be interpreted as the number of times the moment is measured by a force sensor along the transmission path. A specific example will be set forth below to describe in greater detail the design of the sleeve-supported force sensor and how it is used to measure a force.

Fig. 8 is a schematic diagram illustrating force measurement principles of a three-dimensional force sensor according to an embodiment of the present invention. Fig. 8 shows the coordinate systems {q} and {p} respectively for the surgical instrument and the sleeve. As noted above, the origin of the coordinate system {q} is arranged at the intersection of the axis of rotation of the roll joint in the surgical instrument 3 (i.e., the axis of the shaft 302) and the axis of rotation of the pitch joint. In addition, the x-axis of the coordinate system {q} is defined as being perpendicular to the axis of rotation of the pitch joint 305 and coincident with the axis of the shaft 302 in the initial configuration, the y-axis as being parallel to axis of rotation of the pitch joint, and the z-axis as being determined by the right-hand rule. Here, the "initial configuration" refers to a configuration in which the end effector of the surgical instrument 3 extends straight along the direction of the axis of the shaft 302, with its distal end located farthest away from the yaw joint. Further, the coordinate system {p} is established on the end face of the sleeve 401. $r_p$ represents a vector described the translation, and $^qR$ denotes a matrix describing the rotation, from the coordinate system {p} to the coordinate system {q}. Both of them are known quantities determined by an initial mounting position of the sleeve-supported force sensor. For the sake of computational simplicity, it is desirable to ensure, during assembly, that the coordinate systems {p} and {q} are oriented in the same direction so that $^qR$ is an identity matrix. The following analysis is just based on such an orientation of the coordinate systems. In addition, any rotation angle of the surgical instrument at a front end thereof can be measured by an encoder in the power module 301, making $^qR$ still a known quantity.

As shown in Fig. 8, the hollowed-out feature included in the sleeve 401 includes four compliant elements 4011 that are uniformly arranged in the circumferential direction. The force sensor 400 includes four sensitive elements 402, respectively given the subscripts 1 to 4 in the following description for the purpose of distinguishing one from another.

In the illustrated example, each one of the four sensitive elements 402 are fixedly arranged on the outer surfaces of a respective one of the compliant elements 4011, and the sensitive axes of the four sensitive elements 402 all extend parallel to the axial direction of the sleeve 401.

The four sensitive elements are arranged, with their sensitive axes being oriented in individual directions indicated as $e_1$, $e_2$, $e_3$, $e_4$ in the coordinate system {p}. Since all the sensitive elements 402 are oriented in the axial direction of the sleeve 401, each of their directions corresponds to the vector $(1,0,0)^T$. Therefore, according to equations (1-2) and (1-3), the stresses sensed by the four sensitive elements 402 can be represented in the coordinate system {q} as:

$$\begin{cases} \boldsymbol{f}_1 = \left( f_{x1} \quad 0 \quad 0 \right)^T = f_1 \boldsymbol{e}_1 \\ \boldsymbol{f}_2 = \left( f_{x2} \quad 0 \quad 0 \right)^T = f_2 \boldsymbol{e}_2 \\ \boldsymbol{f}_3 = \left( f_{x3} \quad 0 \quad 0 \right)^T = f_3 \boldsymbol{e}_3 \\ \boldsymbol{f}_4 = \left( f_{x4} \quad 0 \quad 0 \right)^T = f_4 \boldsymbol{e}_4 \end{cases} \qquad (1\text{-}6)$$

where $f_{x1}$, $f_{x2}$, $f_{x3}$, $f_{x4}$ are x-axis components of the stress measured by the first to fourth sensitive elements. It is to be noted that, as the directions of the sensitive axes of these sensitive elements are all parallel to the x-axis, components of the stress measured in the other directions, i.e., the y- and z-axis components, are all zero.

Likewise, the four sensitive elements 402 have respective position vectors in the coordinate systems {p}, indicated as $^p r_1$, $^p r_2$, $^p r_3$, $^p r_4$, which are determined by the positions where the sensitive elements 402 are arranged. The representations of the sensitive elements 402 in the coordinate system {q}, i.e., $r_1, r_2, r_3, r_4$ can be obtained from equation (1-4), which are also representations of points of application of the stresses, although the involved equations are not specified herein. The position vectors $r_1, r_2, r_3, r_4$ are given by:

$$\begin{cases} \boldsymbol{r}_1 = \left( x_1 \quad y_1 \quad z_1 \right)^T = \boldsymbol{r}_p + {}^p \boldsymbol{r}_1 \\ \boldsymbol{r}_2 = \left( x_2 \quad y_2 \quad z_2 \right)^T = \boldsymbol{r}_p + {}^p \boldsymbol{r}_2 \\ \boldsymbol{r}_3 = \left( x_3 \quad y_3 \quad z_3 \right)^T = \boldsymbol{r}_p + {}^p \boldsymbol{r}_3 \\ \boldsymbol{r}_4 = \left( x_4 \quad y_4 \quad z_4 \right)^T = \boldsymbol{r}_p + {}^p \boldsymbol{r}_4 \end{cases} \qquad (1\text{-}7)$$

where $x_1$, $x_2$, $x_3$, $x_4$ are x-axis coordinates of centers of the first to fourth sensitive elements in the coordinate system {q}, $y_1$, $y_2$, $y_3$, $y_4$ are y-axis coordinates of the centers of the first to fourth sensitive elements in the coordinate system {q}, and $z_1$, $z_2$, $z_3$, $z_4$ are z-axis coordinates of the centers of the first to fourth sensitive elements in the coordinate system {q}.

According to the definition of the number of redundant forces along a certain direction made in accordance with equation (1-5), in this embodiment, since each of the x-axial stresses is measured only once during its transmission, $n_x = 1$. Likewise, according to the definition of the number of redundant moments along a certain direction, in this embodiment, the following conditions are also satisfied: $n_y' = 1$, $n_z' = 1$. Thus, the contact force acting on the terminal end of the surgical instrument can be obtained, by combining the equations (1-5), (1-6) and (1-7), as:

$$\begin{pmatrix} \boldsymbol{F}_q \\ \boldsymbol{M}_q \end{pmatrix} = \begin{pmatrix} \boldsymbol{f}_1 + \boldsymbol{f}_2 + \boldsymbol{f}_3 + \boldsymbol{f}_4 \\ \boldsymbol{r}_1 \times \boldsymbol{f}_1 + \boldsymbol{r}_2 \times \boldsymbol{f}_2 + \boldsymbol{r}_3 \times \boldsymbol{f}_3 + \boldsymbol{r}_4 \times \boldsymbol{f}_4 \end{pmatrix} \qquad (1\text{-}8)$$

From this equation (1-8), the force $F_{qx}$ acting on the terminal end of the surgical instrument along the x-axis of the coordinate system {q} (axial force) and the moments $M_{qy}$ and $M_{qz}$ along the y- and z- axes of the coordinate system {q} can be obtained as:

$$\begin{cases} F_{qx} = -f_{x1} - f_{x2} - f_{x3} - f_{x4} \\ M_{qy} = -f_{x1}z_1 - f_{x2}z_2 - f_{x3}z_3 - f_{x4}z_4 \\ M_{qz} = f_{x1}y_1 + f_{x2}y_2 + f_{x3}y_3 + f_{x4}y_4 \end{cases} \qquad (1\text{-}9)$$

As can be seen from equation (1-9), arrangement of the four sensitive elements 402 on the force sensor 400 enables force measurement along one, two or three directions. Specifically, when $z_1$, $z_2$, $z_3$, $z_4$ are all zero, the moment $\boldsymbol{M_{qy}}$ along the y-axis of the coordinate system will be zero. Similarly, when $y_1$, $y_2$, $y_3$, $y_4$ are all zero, the moment $M_{qz}$ along the z-axis of the coordinate system will be zero. As a matter of course, $x_1$, $x_2$, $x_3$, $x_4$ are all none-zero values.

According to this embodiment one or more dimensional force measurement as required can be achieved by first determining coordinates of the sensitive elements in the coordinate system {q} and then determining the positions of the sensitive elements on the sleeve based on the derived coordinates. When one-dimensional contact force measurement is required, y-axis and z-axis coordinates of the sensitive elements may be set to zero. When more, such as two or three, dimensional force measurement is required, y-axis or z-axis coordinates of the sensitive elements, or both, may be set to non-zero values. After that, the sleeve structure can be designed as required so that any undesired portion that is not intended to bear a sensitive element is removed by mechanical processing, thereby forming the hollowed-out feature with the compliant elements 4011 for transmitting a force exerted on the cross-section of the sleeve.

While the measurement principles of the force sensor have been described in detail with reference to the foregoing embodiments, it is a matter of course that the present invention includes, but is not limited to, the above configurations listed hereinabove, and any change made thereto is intended to also fall within the protection scope of the present invention. Other embodiments are available for those skilled in the art in light of the above embodiments.

Further, the force sensor of this embodiment is not limited to the measurement of a three-dimensional force (with a force component in one dimension and moment components in the remaining two dimensions), it may be also applicable to the measurement of a two-dimensional force or even a six-dimensional force (with force components in three dimensions and moment components in the remaining three dimensions). In case of multi-dimensional force measurement, the number of the sensitive elements is at least not lower than the number of dimensions of the contact force. In practice, the process of designing the sensor begins with determination of the number of dimensions of the force to be measured. If the force to be measured is n-dimensional, then at least n independent sensitive elements are necessary. After that, a final force measurement equation (e.g., equation (1-10)) with positions and orientations of the sensitive elements as unknowns is established, and how the sensitive elements are arranged is determined based on the direction of the force to be measured. For example, if the force to be measured is in the x-axis direction, the sensitive elements may be reasonably arranged at positions (with determined x, y, z coordinates) and orientations (with determined $f_x, f_y, f_z$), which ensure that a non-zero force measurement can be calculated in said direction. At least, the compliant elements may be designed so as to have the same positions and orientations as the independent sensitive elements. Presented above are merely some basic requirements. In practical engineering applications, in order for improved structural strength and reliability to be achieved, a hollowed-out feature as defined above may be formed across a circumference, with a plurality of (>=2) sensitive elements working in combination to measure a force along a certain direction.

In summary, the force sensor of the present invention is disposed at the terminal end of the surgical instrument and configured to measure a contact force acting on the terminal end. Moreover, the force sensor is provided with a sleeve connected with the surgical instrument, and the sleeve offers at least one of the following advantages:

First, no clearance occurs between the sleeve and the mechanical structure of the surgical instrument. This is conducive to improved measurement accuracy.
Second, for a surgical operation requiring the use of several surgical instruments, a specific calibration process can be performed on each of the surgical instruments, avoiding the influence of instrument material inconsistency on the measurement.
Third, such sleeve can be used on different surgical instruments which transmits deformations of these surgical instruments. Compared to sensitive elements directly attaching to the terminal ends of the surgical instruments, the use of the sensitive elements with the sleeve can avoid measurement errors that may arise from measurement inconsistency between the different sensitive elements on the different surgical instruments, helping in achieving a higher degree of measurement precision and accuracy.
Fourth, coupling the sensitive elements to the surgical instruments with the sleeves can avoid scrappage of the sensitive elements together with the surgical instruments. The surgical instruments are consumable, while the sensitive elements are reusable. Obviously, scraping the sensitive elements together with the surgical instruments is not an economic practice and will lead to increased usage cost.

In a preferred embodiment of the present invention, a plurality of sensitive elements are arranged on the compliant elements of the sleeve in a predetermined manner, and each of the sensitive elements has predetermined coordinates in the coordinate system for the surgical instrument. Based on the predetermined coordinates, the positions of the sensitive elements on the compliant elements can be determined, thus allowing one or more dimensional measurement of a contact force acting on the terminal end of the surgical instrument. This entails an easier and simpler contact force measurement approach, in which one or more dimensional contact force measurement is achievable only by adjusting the positions of the sensitive element relative to the coordinate system for the surgical instrument.

In the present invention, a surgical robot system is further provided. The surgical robot system includes a slave. The slave includes a robotic arm and the surgical instrument as described above. The surgical instrument is detachably connected to a terminal end of the robotic arm and can be driven thereby to move about a remote center of motion (RCM). The surgical robot system also includes a master and a control unit. The master includes a force indicator for showing an information about the contact force acting on the end effector. The control unit is communicatively connected to both the master and the slave and is configured to obtain the information about the contact force acting on the end effector from the force sensor of the surgical instrument and transmit it to the force indicator.

The force indicator may be an imaging system 5 configured to display information about the force acting on the surgical instrument 3. Alternatively, the force indicator may be a master manipulator 7 equipped with a motor. The master manipulator 7 is configured to, upon receiving information about the force acting on the surgical instrument 3, exert a responsive action on the surgeon's hand so that the surgeon can directly "feel" the force acting on the surgical instrument 3.

The slave also includes an endoscope and an endoscope arm detachably connected to the endoscope. In order to facilitate the surgeon to observe how the surgical instrument is being stressed in the field of view of the endoscope, it is necessary to transform the above-described contact force measurement on the terminal end of the surgical instrument into a coordinate system for the endoscope. In this way, a condition of the contact force acting on the terminal end of the surgical instrument can be observed in the field of view of the endoscope (as a force $^eF$ and a moment $^eM$), allowing the surgeon to make any necessary adjustment to the surgical operation being performed based on the contact force.

By transforming the equivalent resultant force $F_q$ and equivalent resultant moment $M_q$ acting on the surgical instrument 3 to the coordinate system {e} for the endoscope according to equation (1-10), the contact force $F$ acting on the surgical instrument 3 can be described accordingly:

$$\begin{pmatrix} ^eF \\ ^eM \end{pmatrix} = \begin{pmatrix} ^eR & 0 \\ -S^T(^er)^eR & ^eR \end{pmatrix} \begin{pmatrix} F_q \\ M_q \end{pmatrix} \qquad (1\text{-}10)$$

where $^eF$ denotes an equivalent resultant force in the coordinate system {e}; $^eM$, an equivalent resultant moment in the coordinate system {e}; $^eR$, the matrix describing the rotation from the coordinate system {q} to the coordinate system {e} (i.e., the posture of the terminal end of the surgical instrument in the endoscope coordinate system);
$^er = (r_x\ r_y\ r_z)^T$, the position vector from the coordinate system {q} to the coordinate system {e} (i.e., the relative position of the terminal end of the surgical instrument to a terminal end of the endoscope); $(\cdot)^T$, a matrix transposition operator; $S(^er)$, an anti-symmetric matrix corresponding to the vector $^er$. $S(^er)$ is given by:

$$S(^er) = \begin{pmatrix} 0 & -r_z & r_y \\ r_z & 0 & -r_x \\ -r_y & r_x & 0 \end{pmatrix} \qquad (1\text{-}11)$$

In this embodiment, an origin of the coordinate system {e} is positioned at the center of the endoscope's field of view. Specifically, the origin of the coordinate system {e} may be located at the terminal end of the endoscope 4. The coordinate system {e} also has an x-axis, a y-axis and a z-axis. The x-axis of the coordinate system {e} is defined as being normal to a lens surface of the endoscope 4 connected to the robotic arm 2. Optionally, an x-axis positive direction of the coordinate system {e} may point from a proximal end of the endoscope 4 (where it is connected to the robotic arm 2) toward the terminal end of the endoscope. The y-axis of the coordinate system {e} is perpendicular to the x-axis thereof and, similarly, the direction of the z-axis of the coordinate system {e} may be determined by the right-hand rule. More preferably, in case of the endoscope being a stereoscopic one, the y-axis of the coordinate system {e} is determined by a line connecting centers of two lens surfaces of the endoscope. Here, since the coordinate system {e} can be established by any suitable technique known in the art, those skilled in the art will recognize, in light of the disclosure herein, how to establish the coordinate system {e} at the endoscope's terminal end and the coordinate system {q} at the terminal end of the surgical instrument, and how to describe, in the coordinate system {e}, the contact force *F* acting on the surgical instrument in the coordinate system {q} through position and posture transformation between the two coordinate systems.

The description presented above is merely that of a few preferred embodiments of the present invention and does not limit the scope thereof in any sense.

**Claims**

1. A surgical instrument (3), comprising a mechanical structure, a sleeve (401) and a force sensor (400);

   wherein the mechanical structure comprises a shaft (302) and an end effector (303) connected to a terminal end of the shaft (302);
   the sleeve (401) fixedly or detachably sleeves over the terminal end of the shaft (302);
   the force sensor (400) comprises at least one sensitive element (402) and a peripheral measurement module (403) connected to the sensitive element (402), the sensitive element (402) is disposed on the sleeve and configured to obtain a strain information of the sleeve (401);
   the peripheral measurement module (403) is configured to obtain a stress measured by the sensitive element (402) based on the strain information obtained by the sensitive element (402) and obtain both an equivalent resultant force and an equivalent resultant moment of an action force exerted by the shaft (302) on the sleeve (401) based on the stress measured by the sensitive element (402), the position of the sensitive element (402) and the direction of a sensitive axis of the sensitive element (402), thereby obtaining a contact force acting on the end effector (303) of the surgical instrument (3).

2. The surgical instrument (3) of claim 1, wherein the sleeve (401) comprises a hollowed-out feature extending along a circumferential direction, the hollowed-out feature comprising a plurality of compliant elements (4011) and openings each located between adjacent two of the compliant elements (4011), the compliant elements (4011) being configured to connect an upper section of the sleeve (401) above the hollowed-out feature to a lower section of the sleeve (401) below the hollowed-out feature, and
   wherein the at least one sensitive element (402) is arranged on the compliant elements and configured to measure a stress on the compliant elements (4011).

3. The surgical instrument (3) of claim 2, wherein the compliant elements (4011) have a modulus of elasticity that is not higher than a modulus of elasticity of the shaft (302); and/or

   each of the compliant elements (4011) comprises an inner surface, an outer surface and side surfaces, and wherein the at least one sensitive element (402) is arranged on the inner surfaces, outer surfaces or side surfaces of the compliant elements (4011); and/or
   the hollowed-out feature is arranged around an axial middle of the sleeve (401).

4. The surgical instrument (3) of claim 1, wherein the sensitive axis of the sensitive element (402) is arranged to be parallel or perpendicular to an axial direction of the sleeve (401).

5. The surgical instrument (3) of claim 1, wherein the sleeve (401) fixedly sleeves over the terminal end of the shaft (302) by bonding, an interference fit or insertion; and/or

   the sleeve (401) detachably sleeves over the terminal end of the shaft (302) by thread connection, a latch connection or a snap fitting; and/or
   the sleeve (401) has one end flush with the terminal end of the shaft (302).

6. The surgical instrument (3) of claim 1, wherein the peripheral measurement module (403) comprises, communicatively connected to one another in series, a data acquisition unit (4031), a signal conditioning unit (4032) and a calculation and output unit (4033); wherein the data acquisition unit (4031) is configured to acquire signals output from the sensitive element (402), the signal conditioning unit (4032) is configured to condition the signals output from the sensitive element (402), and the calculation and output unit (4033) is configured to perform a calculation based on the conditioned signals to obtain the contact force on the end effector (303).

7. The surgical instrument (3) of claim 2, wherein the stress measured by the sensitive element (402) is given by:

$$f_i = g(\varepsilon_i) = k_i \cdot \varepsilon_i + f(\varepsilon_i) \qquad i = 1, 2, \cdots, n,$$

where $I$ represents an i-th sensitive element (402); $f_i$, a stress measured by the i-th sensitive element (402); $\varepsilon_i$, a strain of the compliant element (4011), on which the i-th sensitive element (402) is arranged, along the direction of the sensitive axis of the i-th sensitive element (402); $g(\cdot)$, a function describing a relationship between the stress and the strain; $k_i$, a stress-strain factor of the compliant element (4011), on which the i-th sensitive element (402) is arranged, along the direction of the sensitive axis of the i-th sensitive element (402); and $f(\varepsilon_i)$, a non-linear compensation term for the stress and the strain.

8. The surgical instrument (3) of claim 7, wherein:

the contact force comprises an equivalent resultant force $\boldsymbol{F}_q$ and an equivalent resultant moment $\boldsymbol{M}_q$ ;
the peripheral measurement module (403) is configured to establish a coordinate system {p} for the sleeve (401) thereon, a coordinate system {q} for the surgical instrument (3) at the terminal end thereof, and descriptors of the position and posture of the coordinate system {p} in the coordinate system {q};
the peripheral measurement module (403) is further configured to obtain a descriptor of the stress measured by the sensitive element (402) in the coordinate system {q} based on the descriptor of the posture of the coordinate system {p} in the coordinate system {q}, a descriptor of the direction of the sensitive axis of the sensitive element (402) in the coordinate system {p} and the stress measured by the sensitive element (402), thereby obtaining the equivalent resultant force $\boldsymbol{F}_q$ ;
the peripheral measurement module (403) is further configured to obtain a descriptor of the position of the sensitive element (402) in the coordinate system {q} based on the descriptors of the position and posture of the coordinate system {p} in the coordinate system {q} and a descriptor of the position of the sensitive element (402) in the coordinate system {p}; and
the peripheral measurement module (403) is further configured to obtain a descriptor of a moment of force acting on the sleeve (401) in the coordinate system {q} based on the descriptor of the stress measured by the sensitive element (402) in the coordinate system {q}, thereby obtaining the equivalent resultant moment $\boldsymbol{M}_q$.

9. The surgical instrument (3) of claim 8, wherein

a descriptor of the stress measured by the sensitive element (402) in the coordinate system {p} is given by:

$$\left( f_{xi}, f_{yi}, f_{zi} \right)^T = f_i \boldsymbol{e}_i = f_i \cdot (e_{xi}, e_{yi}, e_{zi})^T \qquad i = 1, 2, ..., n,$$

where $\boldsymbol{e}_i$ is a unit vector denoting a descriptor of the direction of the sensitive axis of the i-th sensitive element (402) in the coordinate system {p}; $f_i$, the stress measured by the i-th sensitive element (402); $f_{xi}, f_{yi}, f_{zi}$, components of the $f_i$ along axes of the coordinate system {p}; and $e_{xi}, e_{yi}, e_{zi}$, components of $e_i$ along axes of the coordinate system {p}.

10. The surgical instrument (3) of claim 9, wherein

the descriptor of the stress measured by the sensitive element (402) in the coordinate system {q} is given by:

$$\boldsymbol{f}_i = {}^q\boldsymbol{R}\left( f_i \boldsymbol{e}_i \right)$$

where $\boldsymbol{f}_i$ represents a descriptor of the stress measured by the i-th sensitive element (402) in the coordinate system {q}, and ${}^q\boldsymbol{R}$ represents the descriptor of the posture of the coordinate system {p} in the coordinate system {q}, and wherein

the equivalent resultant force $F_q$ is given by:

$$F_q = diag(1/n_x, 1/n_y, 1/n_z) \sum_{i=1}^{n} f_i,$$

where $diag(\cdot)$ represents a diagonal matrix with elements in the vector $\cdot$ as diagonal elements, and $n_x, n_y, n_z$ represent numbers of redundant forces in the directions of the x-axis, y-axis and z-axis, respectively.

11. The surgical instrument (3) of claim 8 or 9, wherein

the descriptor of the position of the sensitive element (402) in the coordinate system {q} is given by:

$$r_i = r_p + {}^q R \, {}^p r_i$$

where $r_i$ is a descriptor of the position of the i-th sensitive element (402) in the coordinate system {q}; ${}^p r_i$, a descriptor of the position of the i-th sensitive element (402) in the coordinate system {p}; ${}^q R$, the descriptor of the posture of the coordinate system {p} in the coordinate system {q}; and $r_p$, a descriptor of the position of the coordinate system {p} in the coordinate system {q}; and
preferably,
the equivalent resultant moment $M_q$ is given by:

$$M_q = diag(1/n_x', 1/n_y', 1/n_z') \sum_{i=1}^{n} r_i \times f_i$$

Where $n_x', n_y', n_z'$ represent the numbers of redundant moments in the directions of the x-axis, y-axis and z-axis, respectively.

12. The surgical instrument (3) of claim 9, wherein:

the hollowed-out feature comprises four compliant elements (4011) that are arranged along the circumferential direction; the force sensor (400) comprises four sensitive elements (402), each arranged on an outer surface of a respective one of the compliant elements (4011); the descriptors $e_1, e_2, e_3, e_4$ of the directions of the sensitive axes of the four sensitive elements (402) in the coordinate system {p} are all $(1,0,0)^T$;
the descriptors of the stress measured by the four sensitive elements (402) in the coordinate system {q} are given as:

$$\begin{cases} f_1 = (f_{x1} \quad 0 \quad 0)^T = f_1 e_1 \\ f_2 = (f_{x2} \quad 0 \quad 0)^T = f_2 e_2 \\ f_3 = (f_{x3} \quad 0 \quad 0)^T = f_3 e_3 \\ f_4 = (f_{x4} \quad 0 \quad 0)^T = f_4 e_4 \end{cases}$$

where $f_{x1}, f_{x2}, f_{x3}, f_{x4}$ respectively represent stress measured by the first to fourth sensitive elements (402) in the direction of an x-axis of the coordinate system {q}; and
the equivalent resultant force $F_q$ is given by:

$$F_q = (f_1 + f_2 + f_3 + f_4).$$

13. The surgical instrument (3) of claim 12, wherein:

$^qR$, the descriptor of the posture of the coordinate system {p} in the coordinate system {q}, is an identity matrix; and the positions of the sensitive elements (402) are measured as respective center positions, which are described in the coordinate system {q} as:

$$\begin{cases} r_1 = \begin{pmatrix} x_1 & y_1 & z_1 \end{pmatrix}^T = r_p + {}^p r_1 \\ r_2 = \begin{pmatrix} x_2 & y_2 & z_2 \end{pmatrix}^T = r_p + {}^p r_2 \\ r_3 = \begin{pmatrix} x_3 & y_3 & z_3 \end{pmatrix}^T = r_p + {}^p r_3 \\ r_4 = \begin{pmatrix} x_4 & y_4 & z_4 \end{pmatrix}^T = r_p + {}^p r_4 \end{cases}$$

where $^p r_1, ^p r_2, ^p r_3, ^p r_4$ are descriptors of the center positions of the four sensitive elements (402) in the coordinate system {p}; $r_p$, the descriptor of the position of the coordinate system {p} in the coordinate system {q}; $x_1$, $x_2$, $x_3$, $x_4$, x-axis coordinates of the center positions of the first to fourth sensitive elements (402) in the coordinate system {q}; $y_1$, $y_2$, $y_3$, $y_4$, y-axis coordinates of the center positions of the first to fourth sensitive elements (402) in the coordinate system {q}; $z_1$, $z_2$, $z_3$, $z_4$, z-axis coordinates of the center positions of the first to fourth sensitive elements (402) in the coordinate system {q}; and $r_1$, $r_2$, $r_3$, $r_4$, descriptors of the positions of the first to fourth sensitive elements (402) in the coordinate system {q}; and
preferably,
the equivalent resultant moment $M_q$ is given as:

$$M_q = \left( r_1 \times f_1 + r_2 \times f_2 + r_3 \times f_3 + r_4 \times f_4 \right)$$

14. A surgical robot system, comprising a slave,
the slave comprising:

a robotic arm; and
the surgical instrument (3) as defined in any of claims 1 to 13,
the robotic arm comprising a terminal end detachably connected to the surgical instrument (3), the robotic arm being configured to drive the surgical instrument (3) to move about a remote center of motion; and
preferably, the slave further comprises:

an endoscope (4); and
an endoscope arm detachably connected to the endoscope (4);
wherein the peripheral measurement module (403) of the surgical instrument (3) is configured to establish a coordinate system {p} for the sleeve (401), a coordinate system {q} for the surgical instrument (3) and a coordinate system {e} for the endoscope (4), and to obtain, from a matrix $^eR$ describing a rotation from the coordinate system {q} to the coordinate system $^er = (r_x \ r_y \ r_z)^T$ {e} and a position vector from the coordinate system {q} to the coordinate system {e}, a descriptor of the contact force acting on the end effector (303) in the coordinate system {e} as:

$$\begin{pmatrix} {}^e F \\ {}^e M \end{pmatrix} = \begin{pmatrix} {}^e R & 0 \\ -S^T({}^e r){}^e R & {}^e R \end{pmatrix} \begin{pmatrix} F_q \\ M_q \end{pmatrix}$$

where $^eF$ denotes a resultant force in the coordinate system {e}; $^eM$, a resultant moment in the coordinate system {e}; $S(^er)$, an anti-symmetric matrix corresponding to the vector $^er$; $F_q$, the equivalent resultant force of the contact force acting on the end effector (303); $M_q$, the equivalent resultant moment of the contact force acting on the end effector (303); and

$$S(^{e}\boldsymbol{r}) = \begin{pmatrix} 0 & -r_{z} & r_{y} \\ r_{z} & 0 & -r_{x} \\ -r_{y} & r_{x} & 0 \end{pmatrix}.$$

**15.** The surgical robot system of claim 14, further comprising a master and a control unit, the master comprising a force indicator;

wherein the control unit is communicatively connected to both the master and the slave; the control unit is configured to obtain an information about contact force acting on the end effector (303) from the force sensor (400) of the surgical instrument (3) and transmit the information to the force indicator, the force indicator is configured to show the information about the contact force acting on the end effector (303).

**Patentansprüche**

**1.** Chirurgisches Instrument (3), umfassend eine mechanische Struktur, eine Hülse (401) und einen Kraftsensor (400);

wobei die mechanische Struktur einen Schaft (302) und einen Endeffektor (303) umfasst, der mit einem Endabschluss des Schafts (302) verbunden ist;
wobei die Hülse (401) fest oder lösbar über den Endabschluss des Schafts (302) gehülst ist;
wobei der Kraftsensor (400) mindestens ein sensitives Element (402) und ein mit dem sensitiven Element (402) verbundenes peripheres Messmodul (403) umfasst, wobei das sensitive Element (402) auf der Hülse angeordnet und ausgebildet ist, eine Dehnungsinformation der Hülse (401) zu erhalten;
wobei das periphere Messmodul (403) ausgebildet ist, eine von dem sensitiven Element (402) gemessene Beanspruchung basierend auf den von dem sensitiven Element (402) erhaltenen Dehnungsinformationen zu erhalten und sowohl eine äquivalente resultierende Kraft als auch ein äquivalentes resultierendes Moment einer Wirkkraft zu erhalten, die von dem Schaft (302) auf die Hülse (401) basierend auf der von dem sensitiven Element (402) gemessenen Beanspruchung, der Position des sensitiven Elements (402) und der Richtung einer sensitiven Achse des sensitiven Elements (402) ausgeübt wird, wodurch eine Kontaktkraft erhalten wird, die auf den Endeffektor (303) des chirurgischen Instruments (3) wirkt.

**2.** Chirurgisches Instrument (3) nach Anspruch 1, wobei die Hülse (401) ein ausgehöhltes Merkmal umfasst, das sich entlang einer Umfangsrichtung erstreckt, wobei das ausgehöhlte Merkmal eine Vielzahl von nachgiebigen Elementen (4011) und Öffnungen umfasst, die jeweils zwischen zwei benachbarten der nachgiebigen Elemente (4011) angeordnet sind, wobei die nachgiebigen Elemente (4011) ausgebildet sind, einen oberen Abschnitt der Hülse (401) über dem ausgehöhlten Merkmal mit einem unteren Abschnitt der Hülse (401) unter dem ausgehöhlten Merkmal zu verbinden, und

wobei das mindestens eine sensitive Element (402) auf den nachgiebigen Elementen angeordnet und ausgebildet ist, eine Belastung auf die nachgiebigen Elemente (4011) zu messen.

**3.** Chirurgisches Instrument (3) nach Anspruch 2, wobei die nachgiebigen Elemente (4011) einen Elastizitätsmodul aufweisen, der nicht höher ist als ein Elastizitätsmodul des Schafts (302) ist; und/oder

wobei jedes der nachgiebigen Elemente (4011) eine Innenfläche, eine Außenfläche und Seitenflächen umfasst, und wobei das mindestens eine sensitive Element (402) auf den Innenflächen, Außenflächen oder Seitenflächen der nachgiebigen Elemente (4011) angeordnet ist; und/oder
wobei das ausgehöhlte Merkmal um eine axiale Mitte der Hülse (401) herum angeordnet ist.

**4.** Chirurgisches Instrument (3) nach Anspruch 1, wobei die sensitive Achse des sensitiven Elements (402) parallel oder senkrecht zu einer axialen Richtung der Hülse (401) angeordnet ist.

**5.** Chirurgisches Instrument (3) nach Anspruch 1, wobei die Hülse (401) fest über den Endabschluss des Schafts (302) durch Verklebung, eine Presspassung oder Einsetzen gehülst ist; und/oder

wobei die Hülse (401) lösbar über den Endabschluss des Schafts (302) durch Gewindeverbindung, eine Rastverbindung oder eine Schnapppassung gehülst ist; und/oder

wobei die Hülse (401) ein Ende aufweist, das bündig mit dem Abschlussende des Schafts (302) ist.

6. Chirurgisches Instrument (3) nach Anspruch 1, wobei das periphere Messmodul (403) kommunikativ miteinander in Reihe geschaltet eine Datenerfassungseinheit (4031), eine Signalaufbereitungseinheit (4032) und eine Berechnungs- und Ausgabeeinheit (4033) umfasst; wobei die Datenerfassungseinheit (4031) ausgebildet ist, von dem sensitiven Element (402) ausgegebene Signale zu erfassen, wobei die Signalaufbereitungseinheit (4032) ausgebildet ist, die von dem sensitiven Element (402) ausgegebenen Signale aufzubereiten, und wobei die Berechnungs- und Ausgabeeinheit (4033) ausgebildet ist, eine Berechnung basierend auf den aufbereiteten Signalen durchzuführen, um die Kontaktkraft auf den Endeffektor (303) zu erhalten.

7. Chirurgisches Instrument (3) nach Anspruch 2, wobei die durch das sensitive Element (402) gemessene Belastung gegeben ist durch:

$$f_i = g(\varepsilon_i) = k_i \cdot \varepsilon_i + f(\varepsilon_i) \qquad i = 1, 2, \cdots, n,$$

wobei $i$ ein i-tes sensitives Element (402) repräsentiert; wobei $f_i$ eine durch das i-te sensitive Element (402) gemessene Beanspruchung repräsentiert; wobei $\varepsilon_i$ eine Dehnung des nachgiebigen Elements (4011), auf dem das i-te sensitive Element (402) angeordnet ist, entlang der Richtung der sensitiven Achse des i-ten sensitiven Elements (402) repräsentiert; wobei $g(\cdot)$ eine Funktion repräsentiert, die eine Beziehung zwischen der Beanspruchung und der Dehnung beschreibt; wobei $k_i$ einen Beanspruchungs-Dehnungs-Faktor des nachgiebigen Elements (4011), auf dem das i-te sensitive Element (402) angeordnet ist, entlang der Richtung der sensitiven Achse des i-ten sensitiven Elements (402) repräsentiert; und wobei $f(\varepsilon_i)$ einen nichtlinearen Kompensationsterm für die Beanspruchung und die Dehnung repräsentiert.

8. Chirurgisches Instrument (3) nach Anspruch 7, wobei:

die Kontaktkraft eine äquivalente resultierende Kraft $\boldsymbol{F}_q$ und ein äquivalentes resultierendes Moment $\boldsymbol{M}_q$ umfasst; wobei das periphere Messmodul (403) ausgebildet ist, ein Koordinatensystem {p} für die Hülse (401) auf derselben, ein Koordinatensystem {q} für das chirurgische Instrument (3) an dem Endabschluss desselben, und Deskriptoren der Position und Lage des Koordinatensystems {p} in dem Koordinatensystem {q} aufzustellen; wobei das periphere Messmodul (403) ferner ausgebildet ist, einen Deskriptor der durch das sensitive Element (402) gemessenen Beanspruchung in dem Koordinatensystem {q} basierend auf dem Deskriptor der Lage des Koordinatensystems {p} in dem Koordinatensystem {q}, einen Deskriptor der Richtung der sensitiven Achse des sensitiven Elements (402) in dem Koordinatensystem {p} und die durch das sensitive Element (402) gemessenen Beanspruchung zu erhalten, wodurch die äquivalente resultierende Kraft $\boldsymbol{F}_q$ erhalten wird; wobei das periphere Messmodul (403) ferner ausgebildet ist, einen Deskriptor der Position des sensitiven Elements (402) in dem Koordinatensystem {q} basierend auf den Deskriptoren der Position und Lage des Koordinatensystems {p} in dem Koordinatensystem {q} und einen Deskriptor der Position des sensitiven Elements (402) in dem Koordinatensystem {p} zu erhalten; und wobei das periphere Messmodul (403) ferner ausgebildet ist, einen Deskriptor eines auf die Hülse (401) wirkenden Kraftmoments in dem Koordinatensystem {q} basierend auf dem Deskriptor der durch das sensitive Element (402) gemessenen Beanspruchung in dem Koordinatensystem {q} zu erhalten, wodurch das äquivalente resultierende Moment $\boldsymbol{M}_q$ erhalten wird.

9. Chirurgisches Instrument (3) nach Anspruch 8, wobei

ein Deskriptor der durch das sensitive Element (402) in dem Koordinatensystem {p} gemessenen Beanspruchung gegeben ist durch:

$$\left(f_{xi}, f_{yi}, f_{zi}\right)^T = f_i \boldsymbol{e}_i = f_i \cdot \left(e_{xi}, e_{yi}, e_{zi}\right)^T \qquad i = 1, 2, \ldots, n,$$

wobei $\boldsymbol{e}_i$ ein Einheitsvektor ist, der einen Deskriptor der Richtung der sensitiven Achse des i-ten sensitiven Elements (402) in dem Koordinatensystem {p} bezeichnet; wobei $f_i$ die durch das i-te sensitive Element (402) gemessene Beanspruchung bezeichnet; wobei $f_{xi}, f_{yi}, f_{zi}$ Komponenten der $f_i$ entlang Längsachsen des Koordinatensystems {p} bezeichnen; und wobei $e_{xi}, e_{yi}, e_{zi}$ Komponenten des $\boldsymbol{e}_i$ entlang der Achsen des Koordinatensystems {p} bezeichnen.

10. Chirurgisches Instrument (3) nach Anspruch 9, wobei

der Deskriptor der von dem sensitiven Element (402) in dem Koordinatensystem {q} gemessenen Beanspruchung gegeben ist durch:

$$f_i = {}^q\boldsymbol{R}\left(f_i\boldsymbol{e}_i\right)$$

wobei $\boldsymbol{f}_i$ einen Deskriptor der Beanspruchung repräsentiert, die durch das i-te sensitive Element (402) in dem Koordinatensystem {q} gemessen wird, und wobei ${}^q\boldsymbol{R}$ den Deskriptor der Lage des Koordinatensystems {p} in dem Koordinatensystem {q} repräsentiert, und wobei
die äquivalente resultierende Kraft $\boldsymbol{F}_q$ gegeben ist durch:

$$F_q = diag(1/n_x, 1/n_y, 1/n_z)\sum_{i=1}^{n} f_i$$
,

wobei $diag(\cdot)$ eine diagonale Matrix mit Elementen in dem Vektor • als diagonale Elemente repräsentiert, und wobei $n_x, n_y, n_z$ entsprechend Anzahlen redundanter Kräfte in den Richtungen der x-Achse, y-Achse und z-Achse repräsentiert.

11. Chirurgisches Instrument (3) nach Anspruch 8 oder 9, wobei

der Deskriptor der Position des sensitiven Elements (402) in dem Koordinatensystem {q} gegeben ist durch:

$$r_i = r_p + {}^q\boldsymbol{R}\,{}^p\boldsymbol{r}_i$$

wobei $\boldsymbol{r}_i$ ein Deskriptor der Position des i-ten sensitiven Elements (402) in dem Koordinatensystem {q} ist; wobei ${}^p\boldsymbol{r}_i$ ein Deskriptor der Position des i-ten sensitiven Elements (402) in dem Koordinatensystem {p} ist; wobei ${}^q\boldsymbol{R}$ der Deskriptor die Lage des Koordinatensystems {p} in dem Koordinatensystem {q} ist; und wobei $\boldsymbol{r}_p$ ein Deskriptor der Position des Koordinatensystems {p} in dem Koordinatensystem {q} ist; und
vorzugsweise,
das äquivalente resultierende Moment $\boldsymbol{M}_q$ gegeben ist durch:

$$M_q = diag(1/n_x', 1/n_y', 1/n_z')\sum_{i=1}^{n} r_i \times f_i$$

wobei $n_x', n_y', n_z'$ entsprechend die Anzahlen der redundanten Momente in den Richtungen der x-Achse, y-Achse und z-Achse repräsentieren.

12. Chirurgisches Instrument (3) nach Anspruch 9, wobei:

das ausgehöhlte Merkmal vier nachgiebige Elemente (4011) umfasst, die entlang der Umfangsrichtung angeordnet sind; wobei der Kraftsensor (400) vier sensitive Elemente (402) umfasst, die jeweils auf einer Außenfläche eines entsprechenden der nachgiebigen Elemente (4011) angeordnet sind; wobei die Deskriptoren $\boldsymbol{e}_1$, $\boldsymbol{e}_2$, $\boldsymbol{e}_3$, $\boldsymbol{e}_4$ der Richtungen der sensitiven Achsen der vier sensitiven Elemente (402) in dem Koordinatensystem {p} alle $(1,0,0)^T$ sind;
wobei die Deskriptoren der von den vier sensitiven Elementen (402) in dem Koordinatensystem {q} gemessenen Beanspruchungen gegeben sind als:

$$\begin{cases} \boldsymbol{f}_1 = \begin{pmatrix} f_{x1} & 0 & 0 \end{pmatrix}^T = f_1 \boldsymbol{e}_1 \\ \boldsymbol{f}_2 = \begin{pmatrix} f_{x2} & 0 & 0 \end{pmatrix}^T = f_2 \boldsymbol{e}_2 \\ \boldsymbol{f}_3 = \begin{pmatrix} f_{x3} & 0 & 0 \end{pmatrix}^T = f_3 \boldsymbol{e}_3 \\ \boldsymbol{f}_4 = \begin{pmatrix} f_{x4} & 0 & 0 \end{pmatrix}^T = f_4 \boldsymbol{e}_4 \end{cases}$$

wobei $f_{x1}$, $f_{x2}$, $f_{x3}$, $f_{x4}$ entsprechend Beanspruchungen repräsentieren, die von den ersten bis vierten sensitiven Elementen (402) in der Richtung einer x-Achse des Koordinatensystems {q} gemessen werden; und wobei die äquivalente resultierende Kraft $\boldsymbol{F}_q$ gegeben ist durch:

$$\boldsymbol{F}_q = \left( \boldsymbol{f}_1 + \boldsymbol{f}_2 + \boldsymbol{f}_3 + \boldsymbol{f}_4 \right).$$

**13.** Chirurgisches Instrument (3) nach Anspruch 12, wobei:

$^q\boldsymbol{R}$, der Deskriptor die Lage des Koordinatensystems {p} in dem Koordinatensystem {q}, eine Einheitsmatrix ist; und
wobei die Positionen der sensitiven Elemente (402) als entsprechende Mittelpositionen gemessen werden, die in dem Koordinatensystem {q} beschrieben werden als:

$$\begin{cases} \boldsymbol{r}_1 = \begin{pmatrix} x_1 & y_1 & z_1 \end{pmatrix}^T = \boldsymbol{r}_p + {}^p\boldsymbol{r}_1 \\ \boldsymbol{r}_2 = \begin{pmatrix} x_2 & y_2 & z_2 \end{pmatrix}^T = \boldsymbol{r}_p + {}^p\boldsymbol{r}_2 \\ \boldsymbol{r}_3 = \begin{pmatrix} x_3 & y_3 & z_3 \end{pmatrix}^T = \boldsymbol{r}_p + {}^p\boldsymbol{r}_3 \\ \boldsymbol{r}_4 = \begin{pmatrix} x_4 & y_4 & z_4 \end{pmatrix}^T = \boldsymbol{r}_p + {}^p\boldsymbol{r}_4 \end{cases}$$

wobei $^p\boldsymbol{r}_1$, $^p\boldsymbol{r}_2$, $^p\boldsymbol{r}_3$, $^p\boldsymbol{r}_4$ Deskriptoren der Mittelpositionen der vier sensitiven Elemente (402) in dem Koordinatensystem {p} sind; wobei $\boldsymbol{r}_p$ der Deskriptor der Position des Koordinatensystems {p} in dem Koordinatensystem {q} ist; wobei $x_1$, $x_2$, $x_3$, $x_4$ x-Achsenkoordinaten der Mittelpositionen des ersten bis vierten sensitiven Elemente (402) in dem Koordinatensystem {q} sind; wobei $y_1$, $y_2$, $y_3$, $y_4$ y-Achsenkoordinaten der Mittelpositionen des ersten bis vierten sensitiven Elemente (402) in dem Koordinatensystem {q} sind; wobei $z_1$, $z_2$, $z_3$, $z_4$ z-Achsenkoordinaten der Mittelpositionen der ersten bis vierten sensitiven Elemente (402) in dem Koordinatensystem {q} sind; und wobei $\boldsymbol{r}_1$, $\boldsymbol{r}_2$, $\boldsymbol{r}_3$, $\boldsymbol{r}_4$, Deskriptoren der Positionen des ersten bis vierten sensitiven Elements (402) in dem Koordinatensystem {q} sind; und vorzugsweise,
das äquivalente resultierende Moment $\boldsymbol{M}_q$ gegeben ist durch:

$$\boldsymbol{M}_q = \left( \boldsymbol{r}_1 \times \boldsymbol{f}_1 + \boldsymbol{r}_2 \times \boldsymbol{f}_2 + \boldsymbol{r}_3 \times \boldsymbol{f}_3 + \boldsymbol{r}_4 \times \boldsymbol{f}_4 \right).$$

**14.** Chirurgisches Robotersystem, umfassend einen Slave,
wobei der Slave umfasst:

einen Roboterarm; und
das chirurgische Instrument (3) nach einem der Ansprüche 1 bis 13,
wobei der Roboterarm einen Endabschluss umfasst, der lösbar mit dem chirurgischen Instrument (3) verbunden ist, wobei der Roboterarm ausgebildet ist, das chirurgische Instrument (3) zum Bewegen um ein entferntes Bewegungszentrum anzutreiben; und
vorzugsweise, der Slave ferner umfasst:

ein Endoskop (4); und

einen Endoskoparm, der lösbar mit dem Endoskop (4) verbunden ist;

wobei das periphere Messmodul (403) des chirurgischen Instruments (3) ausgebildet ist, ein Koordinatensystem {p} für die Hülse (401), ein Koordinatensystem {q} für das chirurgische Instrument (3) und ein Koordinatensystem {e} für das Endoskop (4) aufzustellen, und aus einer Matrix $^e\boldsymbol{R}$, die eine Rotation von dem Koordinatensystem {q} zu dem Koordinatensystem $^e r = (r_x \; r_y \; r_z)^T$ {e} beschreibt, und einem Positionsvektor von dem Koordinatensystem {q} zu dem Koordinatensystem {e} einen Deskriptor der auf den Endeffektor (303) wirkenden Kontaktkraft in dem Koordinatensystem {e} zu erhalten, als:

$$\begin{pmatrix} ^e\boldsymbol{F} \\ ^e\boldsymbol{M} \end{pmatrix} = \begin{pmatrix} ^e\boldsymbol{R} & \boldsymbol{0} \\ -S^T(^e\boldsymbol{r})^e\boldsymbol{R} & ^e\boldsymbol{R} \end{pmatrix} \begin{pmatrix} \boldsymbol{F}_q \\ \boldsymbol{M}_q \end{pmatrix}$$

wobei $^e\boldsymbol{F}$ eine resultierende Kraft in dem Koordinatensystem {e} beschreibt; wobei $^e\boldsymbol{M}$ ein resultierendes Moment in dem Koordinatensystem {e} beschreibt; wobei $S(^e\boldsymbol{r})$ eine antisymmetrische Matrix beschreibt, die dem Vektor $^e\boldsymbol{r}$ entspricht; wobei $\boldsymbol{F}_q$ die äquivalente resultierende Kraft der Kontaktkraft beschreibt, die auf den Endeffektor (303) wirkt; wobei $\boldsymbol{M}_q$ das äquivalente resultierende Moment der Kontaktkraft beschreibt, die auf den Endeffektor (303) wirkt; und wobei

$$S(^e\boldsymbol{r}) = \begin{pmatrix} 0 & -r_z & r_y \\ r_z & 0 & -r_x \\ -r_y & r_x & 0 \end{pmatrix}.$$

15. Chirurgisches Robotersystem nach Anspruch 14, ferner umfassend einen Master und eine Steuereinheit, wobei der Master einen Kraftanzeiger umfasst;

wobei die Steuereinheit sowohl mit dem Master als auch mit dem Slave kommunikativ verbunden ist; wobei die Steuereinheit ausgebildet ist, eine Information über auf den Endeffektor (303) wirkende Kontaktkraft von dem Kraftsensor (400) des chirurgischen Instruments (3) zu erhalten und die Informationen an den Kraftanzeiger zu übertragen, wobei der Kraftanzeiger ausgebildet ist, die Information über die auf den Endeffektor (303) wirkende Kontaktkraft anzuzeigen.

**Revendications**

1. Instrument chirurgical (3) comprenant une structure mécanique, un manchon (401) et un capteur de force (400) ;

la structure mécanique comprenant une tige (302) et un effecteur terminal (303) relié à une extrémité terminale de la tige (302) ;

le manchon (401) s'emmanchant de manière fixe ou amovible sur l'extrémité terminale de la tige (302) ;

le capteur de force (400) comprenant au moins un élément sensible (402) et un module de mesure périphérique (403) relié à l'élément sensible (402), l'élément sensible (402) étant disposé sur le manchon et configuré pour obtenir des informations de déformation du manchon (401) ;

le module de mesure périphérique (403) étant configuré pour obtenir une contrainte mesurée par l'élément sensible (402) sur la base des informations de déformation obtenues par l'élément sensible (402) et pour obtenir à la fois une force résultante équivalente et un moment résultant équivalent d'une force d'action exercée par la tige (302) sur le manchon (401) sur la base de la contrainte mesurée par l'élément sensible (402), de la position de l'élément sensible (402) et de la direction d'un axe sensible de l'élément sensible (402), obtenant ainsi une force de contact agissant sur l'effecteur terminal (303) de l'instrument chirurgical (3).

2. Instrument chirurgical (3) selon la revendication 1, le manchon (401) comprenant une caractéristique évidée s'étendant le long d'une direction circonférentielle, la caractéristique évidée comprenant une pluralité d'éléments conformes (4011) et des ouvertures situées chacune entre deux éléments conformes adjacents (4011), les éléments conformes (4011) étant configurés pour relier une section supérieure du manchon (401) au-dessus de la caractéristique évidée à une section inférieure du manchon (401) au-dessous de la caractéristique évidée, et
l'au moins un élément sensible (402) étant agencé sur les éléments conformes et configuré pour mesurer une contrainte sur les éléments conformes (4011).

3. Instrument chirurgical (3) selon la revendication 2, les éléments conformes (4011) ayant un module d'élasticité qui n'est pas supérieur au module d'élasticité de la tige (302) ; et/ou

chacun des éléments conformes (4011) comprenant une surface intérieure, une surface extérieure et des surfaces latérales, et l'au moins un élément sensible (402) étant agencé sur les surfaces intérieures, les surfaces extérieures ou les surfaces latérales des éléments conformes (4011) ; et/ou
la caractéristique évidée étant agencée autour d'un milieu axial du manchon (401).

4. Instrument chirurgical (3) selon la revendication 1, l'axe sensible de l'élément sensible (402) étant agencé de manière à être parallèle ou perpendiculaire à une direction axiale du manchon (401).

5. Instrument chirurgical (3) selon la revendication 1, le manchon (401) s'emmanchant de manière fixe sur l'extrémité terminale de la tige (302) par collage, ajustement serré ou insertion ; et/ou

le manchon (401) s'emmanchant de manière amovible sur l'extrémité terminale de la tige (302) par un raccord fileté, un raccord à loquet ou un raccord à déclic ; et/ou
le manchon (401) ayant une extrémité qui affleure l'extrémité terminale de la tige (302).

6. Instrument chirurgical (3) selon la revendication 1, le module de mesure périphérique (403) comprenant, reliées les unes aux autres en série de manière communicative, une unité d'acquisition de données (4031), une unité de conditionnement de signaux (4032) et une unité de calcul et de sortie (4033), l'unité d'acquisition de données (4031) étant configurée pour acquérir des signaux émis en sortie par l'élément sensible (402), l'unité de conditionnement de signaux (4032) étant configurée pour conditionner les signaux émis par l'élément sensible (402), et l'unité de calcul et de sortie (4033) étant configurée pour réaliser un calcul basé sur les signaux conditionnés afin d'obtenir la force de contact sur l'effecteur terminal (303).

7. Instrument chirurgical (3) selon la revendication 2, la contrainte mesurée par l'élément sensible (402) étant donnée par :

$$f_i = g(\varepsilon_i) = k_i \cdot \varepsilon_i + f(\varepsilon_i) \quad i=1, 2, \ldots, n,$$

i représentant un ième élément sensible (402) ; $f_i$, une contrainte mesurée par le ième élément sensible (402) ; $\varepsilon_i$, une déformation de l'élément conforme (4011), sur lequel le ième élément sensible (402) est agencé, le long de la direction de l'axe sensible du ième élément sensible (402) ; $g(\cdot)$, une fonction décrivant une relation entre la contrainte et la déformation ; $k_i$, un facteur de contrainte-déformation de l'élément conforme (4011), sur lequel est agencé le ième élément sensible (402), le long de la direction de l'axe sensible du ième élément sensible (402) ; et $f(\varepsilon_i)$, un terme de compensation non linéaire pour la contrainte et la déformation.

8. Instrument chirurgical (3) selon la revendication 7,

la force de contact comprenant une force résultante équivalente $\boldsymbol{F_q}$ et un moment résultant équivalent $\boldsymbol{M_q}$ ;
le module de mesure périphérique (403) étant configuré pour établir un système de coordonnées {p} pour le manchon (401), un système de coordonnées {q} pour l'instrument chirurgical (3) à son extrémité terminale, et des descripteurs de la position et de la posture du système de coordonnées {p} dans le système de coordonnées {q} ;
le module de mesure périphérique (403) étant en outre configuré pour obtenir un descripteur de la contrainte mesurée par l'élément sensible (402) dans le système de coordonnées {q} sur la base du descripteur de la posture du système de coordonnées {p} dans le système de coordonnées {q}, d'un descripteur de la direction de l'axe sensible de l'élément sensible (402) dans le système de coordonnées {p} et de la contrainte mesurée par l'élément sensible (402), permettant ainsi d'obtenir la force résultante équivalente $\boldsymbol{F_q}$ ;
le module de mesure périphérique (403) étant en outre configuré pour obtenir un descripteur de la position de l'élément sensible (402) dans le système de coordonnées {q} sur la base des descripteurs de la position et de la posture du système de coordonnées {p} dans le système de coordonnées {q} et d'un descripteur de la position de l'élément sensible (402) dans le système de coordonnées {p} ; et
le module de mesure périphérique (403) étant en outre configuré pour obtenir un descripteur d'un moment de force agissant sur le manchon (401) dans le système de coordonnées {q} sur la base du descripteur de la contrainte mesurée par l'élément sensible (402) dans le système de coordonnées {q}, obtenant ainsi le moment résultant équivalent $M_q$.

9. Instrument chirurgical (3) selon la revendication 8,

un descripteur de la contrainte mesurée par l'élément sensible (402) dans le système de coordonnées {p} étant donné par :

$$(f_{xi}, f_{yi}, f_{zi})^T = f_i e_i = f_i \cdot (e_{xi}, e_{yi}, e_{zi})^T \ i = 1, 2, \ldots, n,$$

$e_i$ étant un vecteur unitaire représentant un descripteur de la direction de l'axe sensible du ième élément sensible (402) dans le système de coordonnées {p} ; $f_i$, la contrainte mesurée par le ième élément sensible (402) ; $(f_{xi}, f_{yi}, f_{zi})$, les composantes de $f_i$ le long des axes du système de coordonnées {p} ; et $e_{xi}, e_{yi}, e_{zi}$, les composantes de $e_i$ le long des axes du système de coordonnées {p}.

10. Instrument chirurgical (3) selon la revendication 9,
le descripteur de la contrainte mesurée par l'élément sensible (402) dans le système de coordonnées {q} étant donné par :

$$f_i = {}^q R(f_i e_i)$$

$f_i$ représentant un descripteur de la contrainte mesurée par le ième élément sensible (402) dans le système de coordonnées {q}, et ${}^q R$ représentant le descripteur de la posture du système de coordonnées {p} dans le système de coordonnées {q}, et la force résultante équivalente $F_q$ étant donnée par :

$$F_q = diag(1/n_x, 1/n_y, 1/n_z) \sum_{i=1}^{n} f_i$$

,

diag($\cdot$) représentant une matrice diagonale dont les éléments du vecteur $\cdot$ sont des éléments diagonaux, et $n_x$, $n_y$, $n_z$ représentant les nombres de forces redondantes dans les directions de l'axe x, de l'axe y et de l'axe z, respectivement.

11. Instrument chirurgical (3) selon la revendication 8 ou 9,
le descripteur de la position de l'élément sensible (402) dans le système de coordonnées {q} étant donné par :

$$r_i = r_p + {}^q R^p r_i$$

$r_i$ étant un descripteur de la position du ième élément sensible (402) dans le système de coordonnées {q} ; $^p r_i$, un descripteur de la position du ième élément sensible (402) dans le système de coordonnées {p} ; $^q R$, le descripteur de la posture du système de coordonnées {p} dans le système de coordonnées {q} ; et $^r p$, un descripteur de la position du système de coordonnées {p} dans le système de coordonnées {q} ; et, de préférence, le moment résultant équivalent $M_q$ étant donné par :

$$M_q = diag(1/n_x', 1/n_y', 1/n_z') \sum_{i=1}^{n} r_i \times f_i$$

$n_x'$, $n_y'$, $n_z'$ représentant les nombres de moments redondants dans les directions de l'axe x, de l'axe y et de l'axe z, respectivement.

**12.** Instrument chirurgical (3) selon la revendication 9,

l'élément évidé comprenant quatre éléments conformes (4011) qui sont agencés le long de la direction circonférentielle ; le capteur de force (400) comprenant quatre éléments sensibles (402), chacun agencé sur une surface extérieure d'un élément conforme respectif (4011) ; les descripteurs $e_1$, $e_2$, $e_3$, $e_4$ des directions des axes sensibles des quatre éléments sensibles (402) dans le système de coordonnées {p} étant tous $(1,0,0)^T$ ; les descripteurs de la contrainte mesurée par les quatre éléments sensibles (402) dans le système de coordonnées {q} étant donnés comme suit :

$$\begin{cases} f_1 = \begin{pmatrix} f_{x1} & 0 & 0 \end{pmatrix}^T = f_1 e_1 \\ f_2 = \begin{pmatrix} f_{x2} & 0 & 0 \end{pmatrix}^T = f_2 e_2 \\ f_3 = \begin{pmatrix} f_{x3} & 0 & 0 \end{pmatrix}^T = f_3 e_3 \\ f_4 = \begin{pmatrix} f_{x4} & 0 & 0 \end{pmatrix}^T = f_4 e_4 \end{cases}$$

$f_{x1}$, $f_{x2}$, $f_{x3}$, $f_{x4}$ représentant respectivement la contrainte mesurée par les premier à quatrième éléments sensibles (402) dans la direction de l'axe x du système de coordonnées {q} ; et
la force résultante équivalente $\mathbf{F}_q$ étant donnée par :

$$F_q = (f_1 + f_2 + f_3 + f_4).$$

**13.** Instrument chirurgical (3) selon la revendication 12,

$^q R$, le descripteur de la posture du système de coordonnées {p} dans le système de coordonnées {q}, étant une matrice d'identité ; et
les positions des éléments sensibles (402) étant mesurées en tant que positions centrales respectives, qui sont décrites dans le système de coordonnées {q} comme suit :

$$\begin{cases} r_1 = \begin{pmatrix} x_1 & y_1 & z_1 \end{pmatrix}^T = r_p + {}^p r_1 \\ r_2 = \begin{pmatrix} x_2 & y_2 & z_2 \end{pmatrix}^T = r_p + {}^p r_2 \\ r_3 = \begin{pmatrix} x_3 & y_3 & z_3 \end{pmatrix}^T = r_p + {}^p r_3 \\ r_4 = \begin{pmatrix} x_4 & y_4 & z_4 \end{pmatrix}^T = r_p + {}^p r_4 \end{cases}$$

$^p r_1$, $^p r_2$, $^p r_3$, $^p r_4$ étant les descripteurs des positions centrales des quatre éléments sensibles (402) dans le système de coordonnées {p} ; $^r \mathbf{p}$, le descripteur de la position du système de coordonnées {p} dans le système de coordonnées {q} ; $x_1$, $x_2$, $x_3$, $x_4$, les coordonnées de l'axe des x des positions centrales du premier au quatrième élément sensible (402) dans le système de coordonnées {q} ; $y_1$, $y_2$, $y_3$, $y_4$, les coordonnées de l'axe des y des positions centrales des premier à quatrième éléments sensibles (402) dans le système de coordonnées {q} ; $z_1$, $z_2$, $z_3$, $z_4$, les coordonnées de l'axe z des positions centrales des premier à quatrième éléments sensibles (402) dans le système de coordonnées {q} ; et $r_1$, $r_2$, $r_3$, $r_4$, les descripteurs des positions des premier à quatrième éléments sensibles (402) dans le système de coordonnées {q} ; et
de préférence,
le moment résultant équivalent $\mathbf{M}_q$ étant donné comme suit :

$$M_q = (r_1 \times f_1 + r_2 \times f_2 + r_3 \times f_3 + r_4 \times f_4)$$

14. Système de robot chirurgical comprenant un esclave,
l'esclave comprenant :

un bras robotique ; et
l'instrument chirurgical (3) tel que défini selon l'une quelconque des revendications 1 à 13,
le bras robotique comprenant une extrémité terminale reliée de manière amovible à l'instrument chirurgical (3),
le bras robotique étant configuré pour entraîner l'instrument chirurgical (3) afin qu'il se déplace autour d'un centre de mouvement éloigné ; et
de préférence, l'esclave comprenant en outre :

un endoscope (4) ; et
un bras d'endoscope relié de manière amovible à l'endoscope (4) ;
le module de mesure périphérique (403) de l'instrument chirurgical (3) étant configuré pour établir un système de coordonnées {p} pour le manchon (401), un système de coordonnées {q} pour l'instrument chirurgical (3) et un système de coordonnées {e} pour l'endoscope (4), et pour obtenir, à partir d'une matrice $^{e}R$ décrivant une rotation du système de coordonnées {q} vers le système de coordonnées $^{e}r = (r_x \ r_y \ r_z)^T$ {e} et un vecteur de position du système de coordonnées {q} vers le système de coordonnées {e}, un descripteur de la force de contact agissant sur l'effecteur terminal (303) dans le système de coordonnées {e} comme suit :

$$\begin{pmatrix} ^{e}F \\ ^{e}M \end{pmatrix} = \begin{pmatrix} ^{e}R & 0 \\ -S^T(^{e}r)^{e}R & ^{e}R \end{pmatrix} \begin{pmatrix} F_q \\ M_q \end{pmatrix}$$

$^{e}F$ désignant une force résultante dans le système de coordonnées {e} ; $^{e}M$, un moment résultant dans le système de coordonnées {e} ; S($^{e}r$), une matrice antisymétrique correspondant au vecteur $^{e}r$ ; $F_q$, la force résultante équivalente de la force de contact agissant sur l'effecteur terminal (303) ; $M_q$ le moment résultant équivalent de la force de contact agissant sur l'effecteur terminal (303) ; et

$$S(^{e}r) = \begin{pmatrix} 0 & -r_z & r_y \\ r_z & 0 & -r_x \\ -r_y & r_x & 0 \end{pmatrix}.$$

15. Système de robot chirurgical selon la revendication 14, comprenant en outre un maître et une unité de commande, le maître comprenant un indicateur de force ;
l'unité de commande étant reliée de manière communicative au maître et à l'esclave ; l'unité de commande étant configurée pour obtenir des informations sur la force de contact agissant sur l'effecteur terminal (303) à partir du capteur de force (400) de l'instrument chirurgical (3) et transmettre les informations à l'indicateur de force, l'indicateur de force étant configuré pour afficher les informations sur la force de contact agissant sur l'effecteur terminal (303).

Fig. 1

Fig. 2

Fig. 3

Fig. 4a

Peripheral
Measurement
Module

403

| Data Acquisition Unit | → | Signal Conditioning Unit | → | Calculation and Output Unit |
|---|---|---|---|---|

4031                           4032                          4033

Fig. 4b

Fig. 5a

Fig. 5b

Fig. 6a

Fig. 6b

Fig. 7a

Fig. 7b

Fig. 8

**EP 3 766 449 B1**

**Patent documents cited in the description**

- US 8491574 B2
- WO 2009079301 A1
- CN 104764552 A

- US 20090157092 A1
- US 20070151390 A1
- JP 2005103056 A